# EUROPEAN PATENT SPECIFICATION

(11) **EP 2 197 533 B1**
(45) Date of publication and mention of the grant of the patent: **01.04.2015**
(21) Application number: 08830923.2
(22) Date of filing: 15.09.2008
(51) Int. Cl.: A61B 18/04, A61B 18/14

(54) **PROSTATE CANCER ABLATION**
ABLATION VON PROSTATAKREBS
ABLATION DU CANCER DE LA PROSTATE

(30) Priority: 14.09.2007 US 972698 P
(43) Date of publication of application: 23.06.2010
(73) Proprietor: Lazure Technologies, LLC, LaConner, Washington 98257 (US)
(72) Inventor: AZURE, Larry, Laconner Washington 98257 (US)
(74) Representative: Gowshall, Jonathan Vallance
(86) International application number: PCT/US2008/076470
(87) International publication number: WO 2009/036471

(56) References cited:
- US-A- 5 529 574
- US-A- 5 529 574
- US-A1- 2003 060 856
- US-A1- 2003 069 619
- US-A1- 2003 069 619
- US-A1- 2003 225 403
- US-A1- 2005 090 732
- US-A1- 2005 222 623
- US-A1- 2006 118 127
- US-A1- 2006 167 499
- US-A1- 2006 167 499

## Description

### BACKGROUND OF THE INVENTION

The present invention relates generally to electric field delivery to a prostate tissue of a patient. More particularly, the present invention provides systems and methods for delivering alternating current and controlled, mild heating to a prostate tissue of a patient for destruction of cancerous and/or hyperplastic tissue.

The prostate gland is a walnut-sized gland located in the pelvic area, just below the outlet of the bladder and in front of the rectum. It encircles the upper part of the urethra, which is the tube that empties urine from the bladder. The prostate is an important part of the male reproductive system, requiring male hormones like testosterone to function properly, and helps to regulate bladder control and normal sexual functioning. The main function of the prostate gland is to store and produce seminal fluid, a milky liquid that provides nourishment to sperm, and increases sperm survival and mobility.

Cancer of the prostate is characterized by the formation of malignant (cancerous) cells in the prostate. Prostate cancer is the leading cancer related cause of death in men in the United States. There are currently over 2 million men in the United States with prostate cancer, and it is expected that there will be approximately 190,000 new cases of prostate cancer diagnosed, with 28,000 men dying from the disease in 2008.

In addition to risks of morbidity due to prostate cancer, most men over 60 years old experience partial or complete urinary obstruction due to enlargement of the prostate. This condition can originate from prostate cancer, or more typically, from benign prostatic hyperplasia (BPH), which is characterized by an increase in prostate size and cell mass near the urethra.

Common active treatment options include surgery and radiation. Surgery often includes the complete surgical removal of the prostate gland ("Radical Prostatectomy"), and in certain instances the regional lymph nodes, in order to remove the diseased tissue from the body. In some instances, a nerve sparing prostatectomy is attempted in an effort to maintain erectile function in the patient after treatment. Side effects associated with radical prostatectomy can include pain, inflammation, infection, incontinence, shorter penis and impotence.

Radiation therapy is another treatment option for prostate cancer and is characterized by the application of ionizing radiation to the diseased area of the prostate. Ionizing radiation has the effect of damaging a cells DNA and limiting its ability to reproduce. For Prostate Cancer treatment, two methods of radiation therapy include External Beam Radiation Therapy (EBRT) and internal radiation, commonly known as Brachytherapy. EBRT involves the use of high-powered X-rays delivered from outside the body. The procedure is painless and only takes a few minutes per treatment session, but needs to be over extended periods of five days a week, for about seven or eight weeks. During EBRT, the rays pass through and can damage other tissue on the way to the tumor, causing side effects such as short-term bowel or bladder problems, and long-term erectile dysfunction. Radiation therapy can also temporarily decrease energy levels and cause loss of appetite.

Brachytherapy involves the injection of tiny radioactive isotope containing 'seeds' into the prostate. Once positioned in the tissue, the radiation from the seeds extends a few millimeters to deliver a higher radiation dose in a smaller area, causing non-specific damage to the surrounding tissue. The seeds are left in place permanently, and usually lose their radioactivity within a year. Internal radiation also causes side effects such as short-term bowel or bladder problems, and long-term erectile dysfunction. Internal radiation therapy can also temporarily decrease energy levels and cause loss of appetite. It is also common for the implanted seeds to migrate from the prostate into the bladder and then be expelled through the urethra during urination. Most significant, however, is the change in the texture of the prostate tissue over time, making the subsequent removal of the gland, as described above, complicated and difficult as a secondary treatment.

Given the significant side-effects with existing treatments such as radical prostatectomy and radiation therapy, less invasive and less traumatic systems and procedures have been of great interest. One such more minimally invasive system developed in recent years includes so called "Trans-urethral Needle Ablation" or TUNA, which involves passing a radio-frequency (RF) device such as a catheter probe or scope into the urethra for delivery of high frequency energy to the tissue. The RF instruments include electrode tips that are pushed out from the side of the instrument body along off-axis paths to pierce the urethral wall and pass into the prostatic tissue outside of the urethra. High-frequency energy is than delivered to cause high-temperature ionic agitation and frictional heating to tissues surrounding the electrodes. The high-temperature induced in the tissue, e.g., up to 90-100 degrees C or more, is non-specific to cancerous tissue and destroys both healthy and non-healthy tissue.

Another technique developed in recent years for treating BPH is Trans-urethral Microwave Thermo Therapy (or "TUMT"). This technique involves use of a device having a microwave probe or antenna located near its distal end and connected to an external generator of microwave power outside the patient's body. The microwave probe is inserted into the urethra to the point of the prostate for energy delivery and microwave electromagnetic heating. Since the microwave probe delivers substantial heating that can cause unwanted damage to healthy tissues or to the urethra, devices typically make use of a cooled catheter to reduce heating immediately adjacent to the probe. The objective is to carefully balance cooling of the urethra to prevent damage to it by the heating process, while at the same time delivering high temperature heating (greater than 50 degrees C) to the prostatic tissue outside of and at a distance from the urethra. In this procedure, the prostatic tissue immediately around the urethra and the urethra itself are deliberately spared from receiving an ablative level of heating by attempting to keep the temperatures for these structures at less than 50 degrees C. Unfortunately, controlling the tissue heating due to the applied microwave energy is difficult and unintended tissue damage can occur. Further, destruction of tissue beyond the cooled region is indiscriminate, and control of the treatment zone is imprecise and limited in the volume of tissue that can be effectively treated.

Accordingly, there is a continuing interest to develop less invasive devices and methods for the treatment of BPH and prostate cancer that is more preferential to destruction of target tissue and more precisely controllable.

US-A1-2006/167499, US-A1-2003/069619 and US-A-5366490 all relate to devices for treatment of the prostrate.

### BRIEF SUMMARY OF THE INVENTION

The invention is as defined in the claims. The present invention provides systems, devices and related methods for applying electric fields, which can be delivered for preferential and/or controllable cancerous cell destruction and tissue ablation. Methods and devices of the present invention will generally be designed to advance an electrode or plurality of electrodes to a target tissue region and apply an electric field to the target tissue region. The electrode or plurality thereof can be positioned such that the applied electric field radiates throughout the target tissue region, including, for example, where the electric field radiates outwardly and in a plurality of directions, e.g., radially, through the target tissue. In certain embodiments, the energy is applied so as to deliver mild and controlled heating of the target tissue.

Thus, the present invention includes systems and devices, as well as methods for delivering electric fields to prostate tissue. Electric field delivery can include establishing an electric current flow through a target tissue region comprising prostate tissue so as to preferentially ablate or destroy cancerous cells in the target tissue region.

For a fuller understanding of the nature and advantages of the present invention, reference should be made to the ensuing detailed description and accompanying drawings. Other aspects, objects and advantages of the invention will be apparent from the drawings and detailed description that follows.

### BRIEF DESCRIPTION OF THE DRAWINGS

FIG. 1 illustrates a device according to an embodiment of the present invention.
FIGS. 2A through 2C illustrate a device according to another embodiment of the present invention.
FIGS. 3A through 3C illustrate a probe and electrode positioning relative to a target tissue region according to several exemplary embodiments of the present invention.
FIGS. 4A and 4B illustrate a system for delivery of electric fields to a prostate tissue of a patient using a plurality or array of electrodes.
FIGS. 5A and 5B shows a system for delivering electric fields to a prostate tissue of a patient using elongate electrode probes and a guide template device.
FIGS. 6A through 6D illustrate field delivery in a target tissue according to various embodiments of the present invention.
FIG. 7 shows a flow chart illustrating energy delivery and therapeutic treatment of a patient's prostate tissue using differentially activated electrodes of an array.
FIGS. 8A through 8C illustrate exemplary electrode embodiments.
FIG. 9 illustrates transurethral system and imaging system according to an embodiment of the present invention.
FIGS. 10A and 10B a transrectal energy delivery system and positioning of electrodes according to an embodiment t of the present invention.
FIG. 11 includes a diagram illustrating a system according to an embodiment of the present invention.
FIGS. 12A and 12B show study results illustrating tumor loss (Figure 12A) and PSA levels (Figure 12B) following treatment according to one aspect of the present invention.
FIG. 13 shows study results illustrating changes in tumor volume following administration of electrical field therapy according to a method of the present invention.

### DETAILED DESCRIPTION OF THE INVENTION

The present invention provides systems and devices, and related methods for prostate tissue ablation. According to the present invention, an electrode or plurality of electrodes can be introduced into a target tissue region and an electric field applied to the target tissue region for controlled and/or preferential destruction of cancerous cells.

Various probe and electrode configurations and/or arrangements may be selected for use according to the present invention and may depend at least partially on the nature and location of the target area. One embodiment of a probe configuration that has been demonstrated to be particularly effective includes probes or electrode configuration with electrodes positioned such that energy delivery includes generating current fields in a plurality of different directions throughout a treatment volume. Further, electrodes can be activated in a bipolar electrical arrangement, including activation in pairs or group combinations, such that tissue disposed between electrodes or within a treatment volume substantially defined by the electrodes acts as a medium through which current field is established or as a current pathway.

In one example, electrodes of a system include a plurality or array of electrodes that can be differentially activated in distinct groups or pairs for establishing different orientations of current field throughout the target tissue. Electrodes can include a plurality of separately controlled electrodes or groups of electrodes, either physically coupled together (e.g., attached to a housing, deployable from a probe, etc.) or can be uncoupled physically and individually positionable and electrically addressable. In some cases, electrode positioning and activation can be selected to establish a current field that is oriented radially through a inner or center of a treatment volume. For example, a probe can be configured such that an inner or centrally located electrode is surrounded by radially spaced electrodes in a bipolar arrangement, and current flow established between the inner electrode and the outer spaced electrodes. Alternatively, a flow center can be established by defining a volume with positioned electrodes and activating a series of opposing electrodes to establish radial current flow through the volume and to destroy cancerous tissue. Regardless of the precise electrode configuration, in one aspect of the present invention, the applied therapeutic field can be contained substantially within the desired treatment region or volume of the target tissue, with current flowed through the target tissue radially or in a plurality of different directions.

Establishment and application of energy delivery utilizing the described energy parameters and/or field delivery (e.g., orientation) can offer several advantages. First, energy delivery according to the present invention further advantageously allows a more controlled or precise therapeutic energy dose both in terms of delivery of the desired current and resulting effects, as well as more accurate delivery to the target or intended tissue. For example, current flow is established between electrodes in a bipolar arrangement, with current flow established and substantially contained between the spaced electrodes. Further, tissue heating can be more precisely controlled to prevent or minimize excessive heating and/or hot spots that can cause unintended damage to healthy or non-target tissues. For example, energy delivery can be selected (e.g., frequency ranges between about 50 kHz to about 300 kHz) such that tissue heating occurs significantly or predominately due to tissue resistance, limiting or minimizing the high-frictional heating observed at high frequencies (e.g., 500 kHz or greater), the latter of which can include significant tissue temperature gradients throughout the treated tissue, with drastic tissue temperature changes occurring as a function of electrode distance. While heating may occur due to both tissue resistance and frictional heating, with relative reduction of high friction type heating, a more constant and controlled heating between opposing electrodes may be delivered.

In one aspect of the inventive methods, relative electrode positioning can be selected so as to further allow more precise control of the desired effect (e.g., induction of mild hyperthermia) of the applied field on the tissue. Factors such as differential conductive properties and resistance or tissue impedance (e.g., differences in muscle, adipose, vasculature, etc.), as well as differential perfusion of blood through vascularized tissue, can effect the ability to control and/or predict effects of delivered current field through certain tissues and varying tissue volumes. Thus, in one embodiment, distances between activated electrodes can controlled and in some cases confined to shorter distances, such as a few centimeters or less, for improved control and predictability of current effects (e.g., tissue heating, field delivery, field orientation, etc) on the targeted tissue.

Another advantage of the present inventive methods and systems is that energy delivery and application of mild hyperthermia as described has been observed to be surprisingly effective in preferentially damaging and destroying cancerous cells compared to non-cancerous or healthy cells/tissue. Preferential destruction, as described herein, refers to establishing current flow as described with application of hyperthermia, generally below about 50 degrees C, such that cytotoxic effects of treatment are, on average or as a whole, more destructive and/or lethal to cancerous or hyperplastic cells (e.g., cells exhibiting or predisposed to exhibiting unregulated growth) compared to non-cancerous or healthy cells. In some instances, establishing current flow and induction of mild hyperthermia as described herein is remarkably effective in preferentially destroying cancerous cells with limited or no observable damage to non-cancerous tissues.

Furthermore, and without being bound by any particular theory, electrode configuration and field application as described in certain embodiments (e.g., radially and/or in a plurality of different directions) may take advantage of tumor or mitotic cell physiology to increase treatment effectiveness, and can include a more optimal or effective orientation of the applied field with respect to dividing cells of the target region. For example, energy application can be accomplished such that current fields are substantially aligned at some point during energy delivery with division axes of dividing cells (e.g., cancerous cells), thereby more effectively disrupting cellular processes or mitotic events (e.g., mitotic spindle formation and the like). As cancerous cells are dividing at a higher rate compared to non-cancerous cells, field application in this manner may preferentially damage cancerous cells compared to healthy or non-dividing cells. It will be recognized, however, that energy application likely has several or numerous cytotoxic effects on cells of the target region and that such effects may be cumulatively or synergistically disruptive to a target cell, particularly to cells disposed or pre-disposed to unregulated growth (i.e., cancerous cells). Other cytotoxic or disruptive effects of the energy application as describe herein may occur due, for example, to application of mild hyperthermia (e.g., mild heating of tissue between about 40 to 48 degrees C; or less than about 50 degrees C); ion disruption, disruption of membrane stability, integrity or function; and the like.

As discussed above, various electrode or probe configurations can be utilized according to the present invention. In one embodiment, electrodes can include an array of needle electrodes, which can be fixed to common support (e.g., housing) or separately positionable and controlled. Such a plurality or array of electrodes can include a straight-needle array including electrically conductive material such as stainless steel, gold, silver, etc. or combination thereof. An array of straight-needle electrodes can be coupled to a rigid needle support or housing that can ensure correct positioning of each individual needle relative to the others. The needles can be arranged parallel to one another with opposing rows and/or columns of electrodes ensuring the field is delivered to and contained within the target area. Needle length and needle spacing can vary depending on the actual dimensions of the target tissue. Individual needle placement can be guided using imaging (e.g., ultrasound, X-ray, etc.) and relative needle position can be maintained with a rigid grid support (e.g., housing, template, etc.) that remains outside the body. The needle assembly will electrically connect to the control system or module, e.g., via insulated wires and stainless steel couplings.

In another embodiment, a probe can include one or more electrodes that are deployable from an elongate probe housing or catheter. Such embodiments may be particularly useful for treatment of target areas more difficult to access with an array of fixed needles. Such deployable type probes, and others described herein, can be inserted percutaneously through the skin of the patient and into the target tissue. As above, appropriate imaging technology can be used to guide the precise placement of the probe in the target site. In one embodiment, a deployable type probe can include outer polyurethane sheath housing pre-shaped deployable shape memory metal tines and a stainless steel central electrode tip. Conductive surfaces can further be coated with a highly conductive material.

Another embodiment of the probe can include one or more expandable elements (e.g., balloon) that can be individually positioned around a target area or organ and then deployed and "inflated" to achieve maximum surface area and optimal distribution of the therapeutic field. In one example, an electrically active segment of the expandable element can include an electrically conductive material (e.g., silver, gold, etc.) coated or deposited on a mylar balloon. Prior to deployment and inflation, the expandable element can be contained inside a flexible catheter that can be guided to the treatment area. Once the delivery catheter is positioned, the "balloon" can be deployed and expanded via the circulation of fluid through the balloon, which can have a selected or controlled temperature and may act as a heat sink. The therapeutic field can than be delivered via the silver coating on the mylar balloon. Two or more probes deployed in this fashion will serve to contain the field within the treatment area.

Electrodes and probes of the present invention can be coupled to control system or control module designed to generate, deliver, monitor and control the characteristics of the applied field within the specified treatment parameters. In one embodiment, a control system includes a power source, an alternating current (AC) inverter, a signal generator, a signal amplifier, an oscilloscope, an operator interface and/or monitor and a central processing unit (CPU). The control unit can manually, automatically, or by computer programming or control, monitor, and/or display various processes and parameters of the energy application through electrodes and to the target tissue of the patient. While the control system and power source can include various possible frequency ranges, current frequency delivered to target tissue will be less than about 300 kHz, and typically about 50 kHz to about 250 kHz. Frequencies in this range have been observed as effective in precisely controlling the energy application to the target tissue, controlling thermal effects primarily to mild thermal application, and preferentially destroying cancerous cells with limited or no observable damage to non-cancerous tissues.

Energy application according to the present invention can further include mild or low levels of hyperthermia. In some embodiments, small changes/elevations in temperature in the target tissue region may occur, but will typically be no more than about 10 degrees C above body temperature, and may be about 2 degrees to less than about 10 degrees C above body temperature (e.g., normal human body temperature of about 38 degrees C). Thus, local tissue temperatures (e.g., average tissue temperature in a volume of treated tissue) during treatment will typically be less than about 50 degrees C, and typically within a range of about 40-48 degrees C. In one embodiment, average target tissue temperature will be selected at about 42-45 degrees C. As target tissue temperatures rise above about 40-42 degrees C curing treatment, the cytotoxic effects of energy delivery on cancerous cells of the target region are observably enhanced, possibly due to an additive and/or synergistic effect of current field and hyperthermic effects. Where mild hyperthermic effects are substantially maintained below about 48 degrees C, the energy delivery according to the present invention appears to more preferentially destroy cancerous cells compared to healthy or non-cancerous cells of the target tissue region. Where energy delivery induces tissue heating substantially in excess of about 45-48 degrees C (e.g., above about 48-50 degrees C), the preferential cytotoxic effects on cancerous cells may begin to diminish, with more indiscriminate destruction of cancerous and non-cancerous cells occurring. Thus, a significant advantage of treatment methods according to the present invention includes the ability to precisely and accurately control energy delivery and induced hyperthermic effects, such that tissue hyperthermia can be accurately controlled and maintained in a desired temperature range(s)-e.g., temperature ranges selected for more targeted or preferential destruction of cancerous cells compared to non-cancerous cells.

Tissue temperatures can be selected or controlled in several ways. In one embodiment, tissue temperatures can be controlled based on estimated or known characteristics of the target tissue, such as tissue impedance/conductivity, tissue volume, blood flow or perfusion characteristics, specific heat capacity of the tissue, tissue density, and the like, with energy application to the tissue selected to deliver an approximated controlled mild increase in tissue temperature. In another embodiment, tissue temperature can be actively detected or monitored, e.g., by use of a thermosensor feedback unit, during treatment, with temperature measurements providing feedback control of energy delivery in order to maintain a desired target tissue temperature or range. Temperature control measures can include electronics, programming, thermosensors, thermocouples, and the like, coupled with or included in a control unit or module of a system of the invention.

Energy application and induction of hyperthermia in a target tissue region according to the present application can include delivery of various types of energy delivery. As described, application of generally intermediate frequency range (e.g., less than about 300 kHz) alternating current in the RF range has been observed as effective in establishing mild heating and hyperthermia, as well as current fields in a controlled manner so as to provide a cytotoxic effect, and in some instances, a preferential destructive effect to cancerous cells of a target tissue volume/region. It will be recognized, however, that additional energy applications and/or ranges may be suitable for use according to the present invention, and that systems and methods of the present invention may be amenable to use with other or additional energy applications. For example, energy application can include current flow having frequencies found generally in the RF range, as well as microwave range, including higher frequencies such as 300-500 kHz and above, and may further be amenable to use with direct current applications. Applied current can be pulsed and/or continuously applied, and energy delivery can be coupled with a feedback-type system (e.g., thermocouple positioned in the target tissue) to maintain energy application and/or tissue heating in a desired range. Methods of the present invention can include any one or more (e.g., combination) of different energy applications, induced temperatures, etc. as described herein.

In certain embodiments, particularly where energy application is selected for lower power delivery/ablation, the control system can be designed to be battery powered and is typically isolated from ground. In such an embodiment, AC current is derived from the integrated power inverter. An intermediate frequency (e.g., less than 300 kHz; or about 50 kHz to about 250 kHz) alternating current, sinusoidal waveform signal is produced from the signal generator. The signal is then amplified, in one non-limiting example, to a current range of 5 mA to 50 mA and voltage of up to 20 Vrms per zone. Field characteristics including waveform, frequency, current and voltage are monitored by an integrated oscilloscope. Scope readings are displayed on the operator interface monitor. An integrated CPU monitors overall system power consumption and availability and controls the output of the signal generator and amplifier based on the treatment parameters input by the operator. The operator can define treatment parameters to include maximum voltage, maximum current or temperature, maximum power, and the like. In another embodiment, the applied field can be cycled on and off, e.g., at a high rate, to keep the temperature relatively constant and with the duty cycle (e.g., on time - off time) adjusted to accurately control temperature.

Imaging systems and devices can be included in the methods and systems of the present invention. For example, the target tissue region can be identified and/or characterized using conventional imaging methods such as ultrasound, computed tomography (CT) scanning, X-ray imaging, nuclear imaging, magnetic resonance imaging (MRI), electromagnetic imaging, and the like. In some embodiments, characteristics of the tumor, including those identified using imaging methods, can also be used in selecting ablation parameters, such as energy application as well as the shape and/or geometry of the electrodes or array of electrodes. Additionally, these or other known imaging systems can be used for positioning and placement of the devices and/or electrodes in a patient's tissues.

A target tissue will include prostate tissue or tissue including cancerous prostate cells and/or hyperplastic tissue of the prostate or prostate region. Thus the present invention includes delivery of electrical fields and ablation therapy to a target tissue including prostate tissue by making use of the techniques, systems and probes described herein. Prostate tissue can be accessed for delivery of electrical fields as described herein can by using a variety of methods. For example, prostate tissue access can include any of a variety of currently know access/surgical methods used for existing prostate treatment techniques, which will be modified for delivery of the ablation treatment as described herein. Surgical access can include, for example, techniques commonly employed for surgical intervention for prostate cancer that involves radical prostatectomy via an abdominal (retropubic) or perineal approach, or various robotic methods. Rather than removing the prostate tissue via surgical excision, however, electrodes of a probe according to the present invention can by positioned in the target tissue including prostate cells/cancerous cells and current applied to the tissue as described herein. While the present techniques can provide an alternative therapy to other techniques such as radical prostatectomy, in some cases other surgical techniques can optionally be used in addition or conjunction with the ablation techniques of the present invention. For example, treatment may first be delivered via ablation therapy of the present invention and followed (e.g., at a later time) by other surgical techniques, such as partial or entire prostatectomy. Such an approach may in some instances improve outcomes and/or reduce complications commonly associated with other treatments such as surgical removal of the prostate, e.g., by reducing the amount of tissue in need of surgical removal.

Other known prostate tissue access techniques besides more traditional surgical access can be employed in delivery of ablation therapy of the present invention. For example, surgical techniques commonly used in hyperthermic ablation methods can be employed for ablation therapy according to the present invention, including various transurethral access methods, such as those commonly employed in transurethral needle ablations, transurethral microwave ablation, ultrasound (high-intensity focused ultrasound), electrical vaporization (transurethral electrical vaporization of the prostate), and the like. Various other techniques, including minimally invasive techniques, can be employed, including laparoscopic techniques (e.g., percutaneous puncture/laparoscopic techniques), transrectal access or puncture, and the like. Various monitoring techniques can be used in conjunction with ablation. For example, imaging systems and devices (see, e.g., as described below), diagnostic monitoring (e.g., prostate-specific antigen (PSA) testing), etc. can be used to evaluate and/or monitor disease state and/ or treatment progression.

Thus, energy delivery probes, according to the present invention, can be advanced and positioned according to various prostate tissue access techniques. Methodologies and access techniques, as noted above, can include without limitation open surgical techniques, laparoscopic or minimally invasive surgical access, puncture and/or advancement (e.g., percutaneous puncture) of probes and/or electrodes through the perineum, as well as transurethral and/or transrectal access. Exemplary probe configurations and positioning, according to certain embodiments of the present invention, are generally described further below.

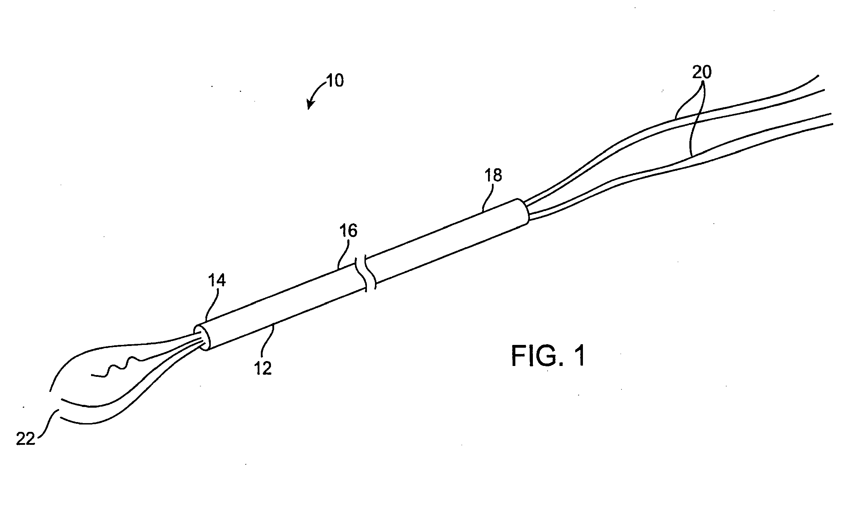

Referring to FIG. 1, a device according to an embodiment of the present invention is described. The device 10 includes a delivery member 12 having a distal portion 14 and a proximal portion 16. The device 10 further includes a proximal portion 18 of the device that can be coupled (e.g., removably coupled) to the delivery member 12. Additionally, the device 10 can include conductive cables 20 electrically coupled to an energy source (not shown). The device includes a plurality of electrodes 22 at the distal portion 14 of the delivery member 12. The electrodes 22 can be positioned or fixed, for example, at the distal end of the delivery member 12 or positionable and deployable from a lumen of the delivery member 12 and retractable in and out of the distal end of the delivery member 12. The electrodes 22 can include a non-deployed state, where the electrodes 22 can be positioned within a lumen of the delivery member 12, and a deployed state when advanced from the distal end of the delivery member 12. Electrodes 22 are advanced out the distal end and distended into a deployed state substantially defining an ablation volume.

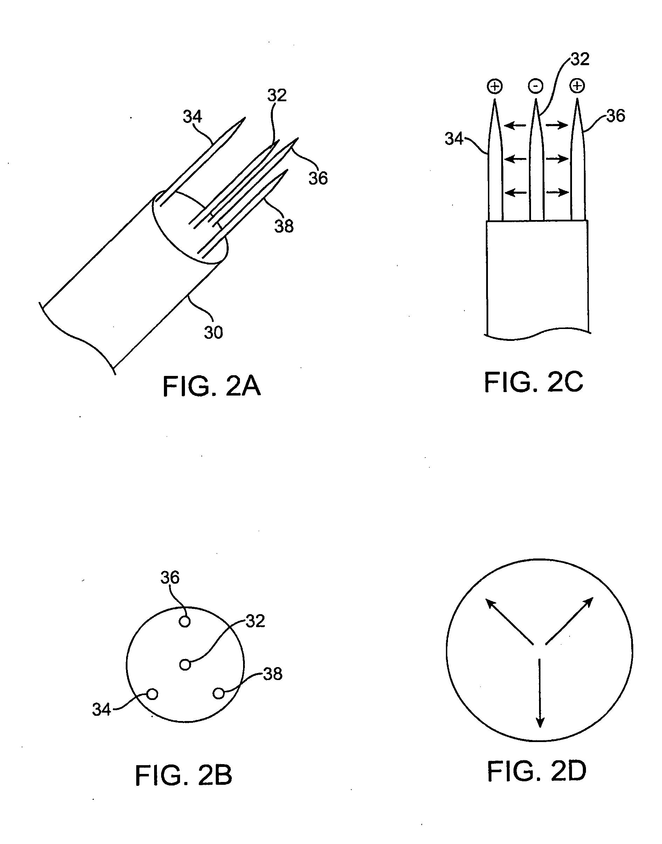

In another embodiment, a probe can include a plurality of needle electrodes fixed to or positioned on a body or housing of a device. FIGS. 2A through 2C show a device having a plurality of electrodes coupled to a housing, according to another embodiment of the present invention. As shown, the device 30 includes a plurality of electrodes extending from the distal portion (e.g., housing) of the device. FIG. 2A shows a three dimensional side view of the device having the plurality of electrodes. FIG. 2B shows a top view of the device illustrating the electrode arrangement. The plurality includes a centrally positioned electrode 32 and outer electrodes 34, 36, 38 spaced laterally from the central electrode 32. The illustrated electrodes include substantially linear needle-like portions or needle electrodes. The electrodes extend from the distal portion of the device and are oriented to be substantially parallel with the longitudinal axis of the device 30. Additionally, each electrode is substantially parallel with other electrodes of the plurality. The plurality of electrodes substantially define the ablation volume, with the outer electrodes 34, 36, 38 substantially defining a periphery of the ablation volume and the electrode 32 positioned within or at about the center point of the defined periphery. Each of the electrodes can play different roles in the ablation process. For example, there can be changes in polarity and/or polarity shifting between the different electrodes of the device. As with other devices of the invention, electrodes can be electrically independent and separately addressable electrically, or two or more electrodes can be electrically connected, for example, to effectively function as one unit. In one embodiment, for example, outer electrodes 34, 36, 38 can be electrically connected and, in operation, include a polarity different from that of the inner electrode 32. As illustrated in FIG. 2C the electrodes 32 and 34, 36 of the device can include opposing charges (e.g., bipolar). In such an instance, the applied electrical current can provide an electrical field, as illustrated by the arrows, extending radially outward from the central electrode 32 and toward the peripherally positioned or outer electrode(s) 34, 36. Figure 2D illustrates the concept of a current flow center, where current flow is established through about a center location of a treatment volume.

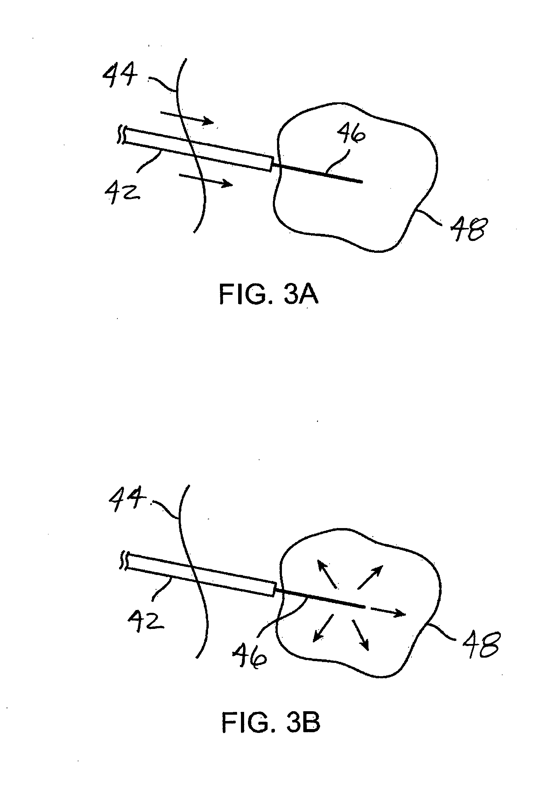

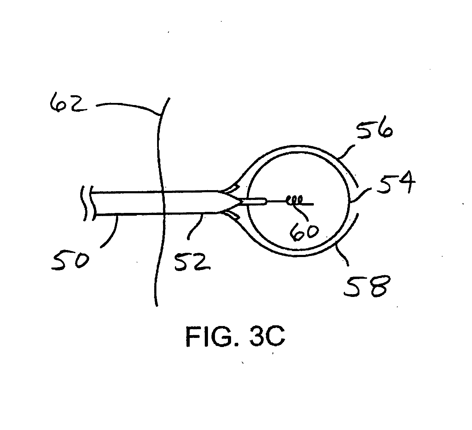

In use, as shown in Figures 3A through 3C, a device 42 of the present invention can be advanced through the patient's tissue 44, and an electrode 46 of the device 42 positioned within a target tissue region 48 (e.g., prostate tissue). Once the electrode 46 is positioned in the target tissue region 48, electrical current is delivered to the target tissue region 48 or treatment region. As the electrode 46 is positioned within the target tissue region 48, the applied electrical current can provide an electric field that radiates outward and in a plurality of directions. In a bipolar mode embodiment, outer electrodes substantially defining an ablation volume can function as return electrodes, or complete a circuit with an electrode(s) positioned within the ablation volume, with applied current flowing through tissue of the target region positioned between the outer electrodes and electrode(s) positioned within the ablation volume. Figure 3C shows use of a deployable electrode device 50 of the present invention according to another embodiment of the present invention. As described above, the device 50 is advanced through the patient's tissue 62 and the delivery member 52 positioned proximate to the target tissue region 54. Once the delivery member 52 is positioned, a plurality of electrodes 56, 58, 60 can be deployed from the delivery member 52. Outer electrodes 56, 58 are deployed within or around the perimeter of the target tissue region 54, e.g., at about the margin of the target tissue region (e.g., tumor margin) and substantially define the ablation volume or target region. The inner electrode 60 is positioned within the ablation volume.

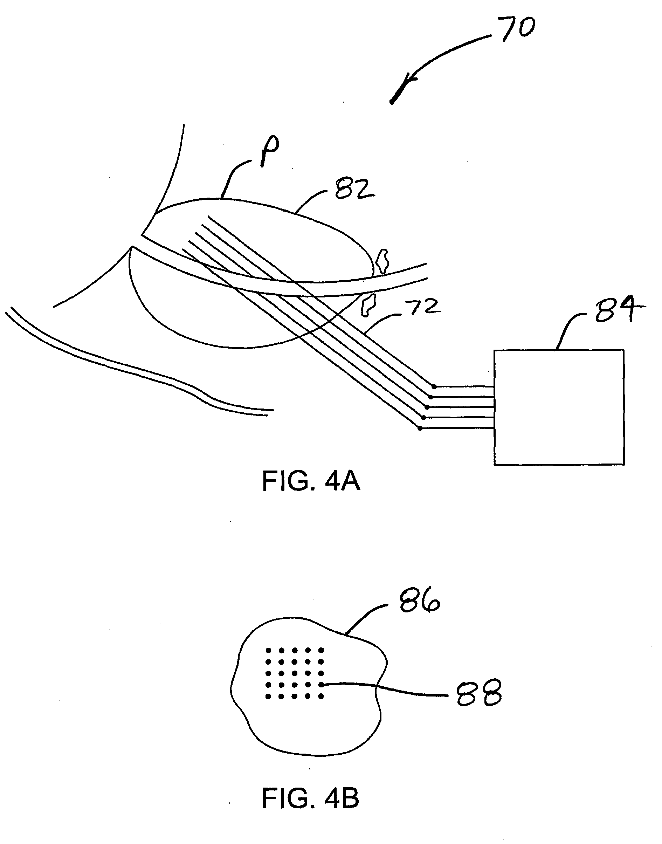

In another embodiment of the present invention, systems and methods can include a plurality of electrodes (e.g., needle electrodes) that can be individually advanced and positioned in the target/prostate tissue, and electrically activated for energy delivery (see, e.g., Figure 4 below). In such an embodiment, an array of electrodes can be advanced through the perineum of the patient and electrically activated (e.g., differentially activated) to deliver current field in a plurality of different directions. An array or plurality as described can include various numbers of electrodes, and the selected number can depend, at least partially, on factors such as target tissue characteristics, treatment region, needle size, and the like. An array can include a few to several dozen electrodes. In one example, an array can include about a few electrodes to about a dozen or more (e.g., 10-100, any number therebetween, or more) electrodes for positioning in the target tissue region.

A system and method for delivering electric fields according to the present invention is described with reference to Figures 4A and 4B. The system 70 includes an electrode array 72 that can be positioned in a target tissue 82 (e.g., prostate tissue). Elongated needle electrodes in electrode array 72 will include a distal portion and a proximal portion. The proximal portion of each electrode will be electrically connected to a system control unit or module 84, which includes electronics, storage media, programming, etc., as well as a power generator, for controlled delivery of selected electrical fields to the target tissue 82. In use, electrode array 72 will be advanced through the prostate tissue (P) and to a desired position, as shown in Figure 4A. Electrode positioning can include, for example, insertion and advancement through the skin and through the perineum of the patient. Electrode positioning and arrangement within the target tissue 82 can be precisely controlled and may occur under the guidance of tissue imaging methodology (e.g., ultrasound imaging, X-ray, CT, etc.). Figure 4B illustrates a cross-section view of a target tissue 86 having a plurality of positioned needle electrodes 88.

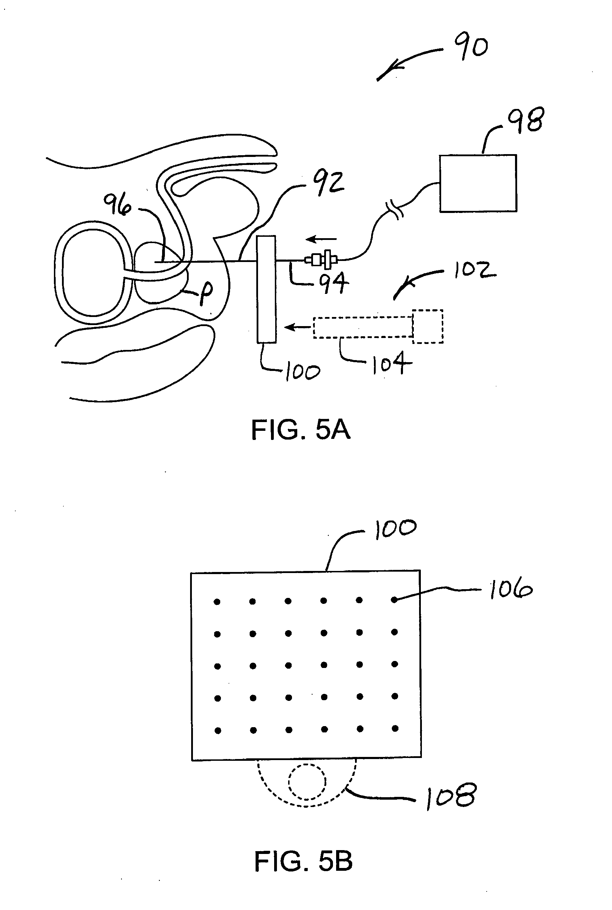

A system for implementing a method according to the present invention is shown in Figures 5A and 5B, including transperineal access and positioning of electrodes using a positioning template, and delivery of electric fields to the prostate tissue. Referring to Figure 5A, the system 90 includes a plurality of elongated probes such as probe 92, having a proximal portion 94 and a distal portion 96. The distal portion 96 includes a portion configured for delivery of the electrical field when positioned in the prostate tissue (P). The probe can be advanced through the skin and through the perineum of the patient so that the distal portion is positioned in the target area (e.g., prostate tissue (P)) of the patient. The proximal portion 94 of the probe 92 will be electrically connected to a system control unit 98, as above, which can include electronics, storage media, programming, etc., as well as a power unit, for controlled delivery of selected electrical fields to the target tissue. As illustrated, the system 90 can optionally include a guide template 100 for controlled placement and positioning of the probe 92 in the tissue of the patient. The system 90 can further include an imaging device/system 102, which can include imaging systems described further herein, which may be used for guidance and placement of the probe 92. For example, the device 102 can include a distal portion 104 including electronics and imaging components (e.g., ultrasonic scanning transducer), which can be inserted in the patient's rectum (R) and positioned against the rectal wall near the prostate (P). An exemplary imaging device 102 can include those commonly used for diagnostic medicine, such as ultrasonic imaging devices provided by Accuson, Inc. (Mountain View, CA). The guide template 100 can optionally be designed for coupling with the imaging device 102, such that guide template 100 and the imaging device 102 form a single stable assembly.

A guide template 100, according to an exemplary embodiment of the present invention, is described in further detail with reference to Figure 5B. The template 100 includes a plurality of guides 106 (e.g., guide holes or via) through which the probes 92 can be inserted and distal portions of the probes advanced through the patient's tissue in a controlled manner. Guides 106 can be disposed to form an array on the template 100 and can be specifically sized to match or substantially match the received probes 92, such that probes are positioned and held in the desired position. As noted above, the template 100 can optionally be designed for assembly with an imaging device 102, and may include an imaging device receiving portion 108 through which the imaging device can be inserted.

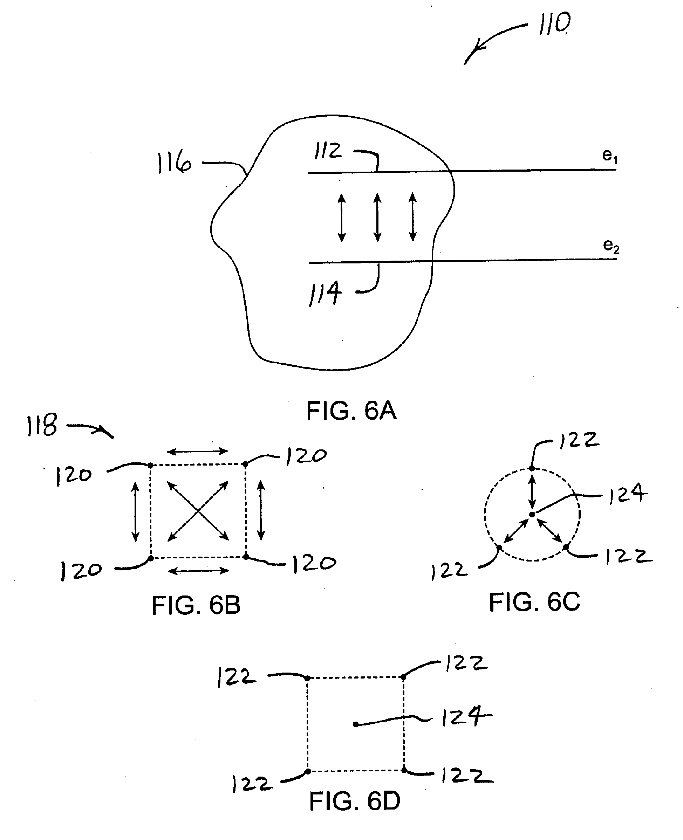

Electrode positioning and energy delivery is further described with reference to Figures 6A through 6D. As described above with reference to Figures 4 and 5, the present invention can include insertion and positioning of a plurality or array of individual electrodes, with the electrodes being controlled individually or in groups and activated to deliver current field to the target tissue in a plurality of different orientations and directions. Electrodes can be differentially activated in various different pairs or groups such that the desired electric field is delivered to the target tissue in a plurality of different directions. Figure 6A illustrates use of an electrode pair as a basic field delivery unit 110 of the electrode array, according to one example. As shown, distal portions 112, 114 of two electrodes (e₁ and e₂) of a plurality positioned in a target tissue 116 and activated as an electrode pair or circuit, with the applied current substantially contained between the two. Thus, electrodes can be activated in a bipolar configuration, with current flowing between electrodes (e.g., between e₁ and e₂) and the tissue between the electrodes acting as a flow medium or current pathway between the electrodes. Controlled activation of pairs or relatively small groups of electrodes in this manner allows more precise control of the current applied to the tissue, containment of the applied field to the desired location, as well control of heating or limited temperature increase in the target tissue 116. Several factors may lend to improved control of therapeutic effects of the delivered fields according to the present invention. First, as discussed above activating electrode in pairs or groups in a bipolar configuration or so as to form a circuit allows the applied field to substantially be contained within the volume defined by the positioned electrodes. Second, energy delivery can be selected (e.g., frequency ranges between about 50 kHz to about 300 kHz) such that tissue heating occurs substantially due to tissue resistance, relative to the frictional heating observed at high frequencies (e.g., 500 kHz or greater). High frequency/high friction type heating is typically characterized by significant tissue temperature gradients throughout the treated tissue, with substantially higher tissue temperatures occurring near the electrode.

Another advantage of methods using the described electrode array or plurality of the present invention is that relative electrode positioning can be limited to smaller distances so as to further allow more precise control of the desired effect of the applied field on the tissue. Factors such as differential conductive properties and resistance or tissue impedance (e.g., differences in muscle, adipose, vasculature, etc.), as well as differential perfusion of blood through vascularized tissue, can limit the ability to control and/or predict effects of delivered current field traversing larger distances through tissue. In the present invention, distances between activated electrodes can be limited to shorter distances, such as a few centimeters or less, for improved control and predictability of current effects (e.g., tissue heating, field delivery, orientation, etc) on the targeted tissue. Thus, activated electrodes in a pair or group can be spaced less than about 4 cm apart. For example, adjacent electrodes of a pair or group will typically be positioned within about 0.1 cm to about 2 cm of each other. Distances of about 0.5 cm have been shown to be particularly effective in providing controlled and predictable field delivery, controlled tissue heating, as well as substantial therapeutic effect.

As described above, a plurality of electrodes can be positioned in the target tissue of the prostate of a patient and the electrodes can be activated in pairs or groups to deliver the therapeutic current field to destroy cancerous tissue. A particular electrode of an array need not be confined to a single unit, but can be activated at different times in conjunction with different electrodes of the plurality. For example, differential activation can include activating a specific or selected series of electrode groups in a particular or predetermined order. In one embodiment, a series of selected pairs or groups can be activated in seriatim and/or in a predetermined order, with activation control typically being determined by operation or instructions (e.g., programming) of a control system or module. Sequences of group activations can be controlled and repeated, manually or by automation, as necessary to deliver an effective or desired amount of energy.

Such differential activation advantageously allows delivery of fields throughout the target tissue and in a plurality of different directions. As shown in Figure 6B, a simple four electrode grouping 118 of an array can be differentially activated in pairs, with each different pair of electrodes 120 providing a different field delivery and orientation (possible field flow/orientations are illustrated by arrows). While activation of electrodes in discrete pairs provides simplicity, electrodes can be activated in groups for more diverse field orientation and deliver. For example, a delivery unit can include a centrally located electrode surrounded by spaced electrodes, with the applied field extending between the central electrode and the outer spaced electrodes. In this manner, the outer electrodes can essentially define an ablation volume with the inner/central electrode positioned within the volume. Field delivery in this way is advantageously controlled and substantially contained within the ablation volume. Figures 6C and 6D illustrate exemplary electrode positioning including outer electrodes 122 and an inner or centrally located electrode 124. Electrode positioning will not be limited to any particular configuration, and various arrangements will be possible.

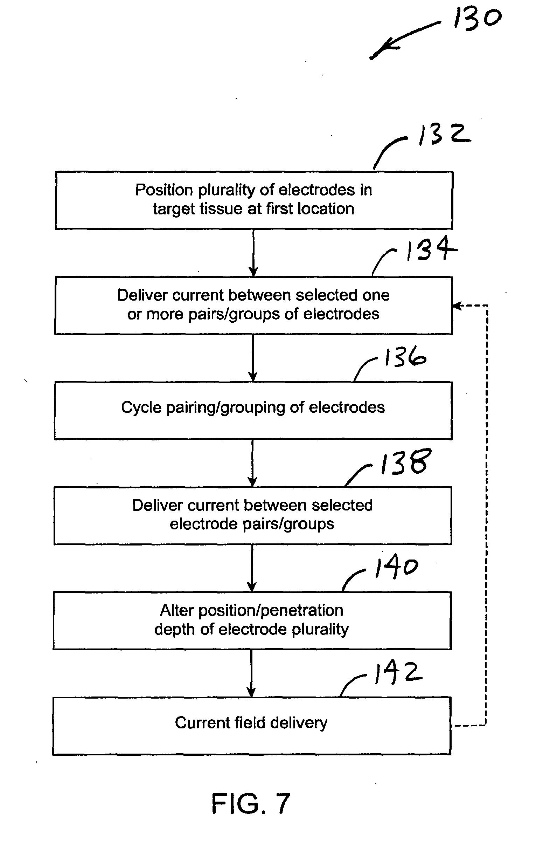

Figure 7 schematically illustrates a method 130 encompassed by the present invention. As in the embodiments described in Figures 4 and 5, for example, a system of the present invention can include a plurality of electrodes that can be positioned in the target tissue or prostate tissue of a patient, with selected current delivery and application to the tissue occurring by differential activation of various groups or pairs of electrodes. Thus, a method of the present invention, as shown in Figure 7, can include positioning a plurality of electrodes in a target tissue of a patient at a first or initial treatment location (Step 132). The plurality can be positioned entirely within the patient's prostate tissue or may include positioning of at least some electrodes of the plurality at or beyond the prostate tissue margin. In some cases, positioning for initial current delivery may include advancing electrodes through the perineum of the patient and to a distal most portion of the prostate tissue nearest the patient's bladder. Electrode advancement and positioning may be aided or guided by tissue imaging techniques. Once the desired initial treatment positioning of the electrodes has been achieved, initial field delivery can occur. As described above (see, e.g., Figures 6A through 6D), current can be delivered to the target region of the tissue in a plurality of different directions or current orientations be differentially selecting between and activating different pairs/groups of electrodes. Different groups or pairs of electrodes can be activated individually or in sequence, or a plurality of different groups can be activated simultaneously. For example, treatment can include selecting a first grouping or pairing of electrodes for activation, and delivering current between the selected pairs/groups (Step 134). Current delivery can be cycled through different pairings or groupings of electrodes by discontinuing current delivery through the first selected grouping, and selecting a second or subsequent grouping for activation (Step 136). Following cycling or selecting a different subsequent grouping, current is delivered between the next selected electrode pair/group (Step 138). Following current delivery at the initial treatment positioning of the electrodes, the one or more of the plurality can be removed from the tissue or the position of the electrodes altered for a next phase of current delivery (Step 140). For example, electrodes can be withdrawn a short distance in a proximal direction to alter the electrode penetration depth for a next phase of current field delivery (Step 142). Current delivery and electrode re-positioning can be repeated until the desired volume of the tissue has been treated.

Treatment time according to the present invention can be selected based on a variety of factors, such as characterization of the tissue, energy applications selected, patient characteristics, and the like. Energy application to a target tissue region during treatment according to the present invention can be selected from a few minutes to several hours. Though, effective treatment is expected to occur in about 5 minutes to 90 minutes. Effective preferential destruction of cancerous prostate cells has been observed in less than one hour, and in many cases about 15-30 minutes of energy application. Treatment can include a single energy delivery period or dose, or multiple phases or doses of energy application. As described above, electrodes can be positioned in a first location and energy delivered, then moved to subsequent location(s) for subsequent energy delivery. Treatment can occur in phases or repeated, and/or may be coupled with additional or alternative treatments or energy delivery methods.

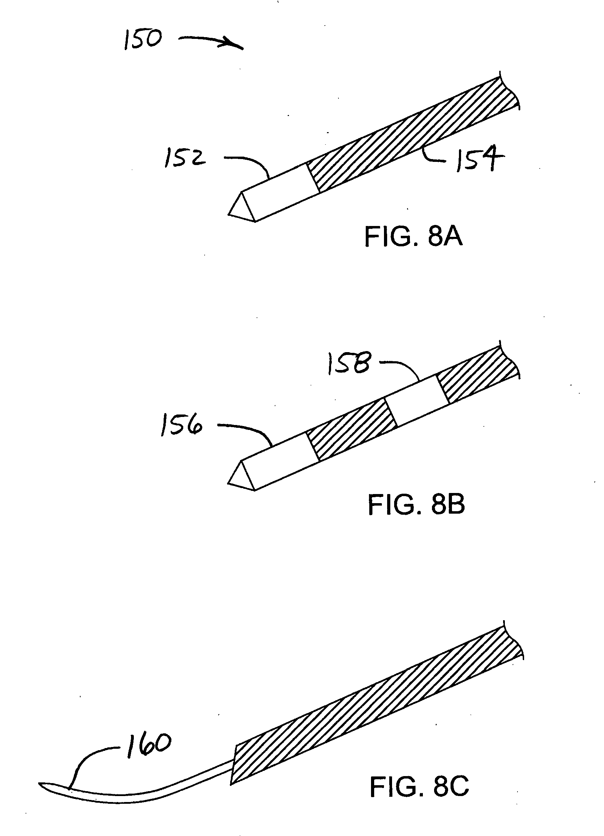

As described above, electrodes will include a distal portion having an electrically active region for delivering the desired current field to the target tissue. Various electrode configurations and designs can be utilized and the current invention is not limited to any particular electrode design. Electrodes, for example, can be differentially insulated such that current delivery occurs at a non-insulated or thinly insulated region of the electrode. Figure 8A illustrates a straight needle electrode 150 having an electrically active region 152 and a region 154, which is non-electrically active. The needle 150 can include an electrically conductive material (e.g., stainless steel, silver, gold, etc.) having an insulating coating on region 154 and non-insulated on the active region 152. Electrodes can include a single active region or a plurality of active regions, as shown in Figure 8B having active regions 156, 158. In addition to more rigid straight needle type electrodes, electrodes can include a deployable element that can be retractable and positioned within a lumen of a catheter-type device, as shown in Figure 8C. The electrode element 160 can be curved (as shown) or can be substantially straight or linear. Various needle/electrode sizes and/or configurations may be utilized, and can include, without limitation, needles ranging from about 15 to about 27 gauge in size.

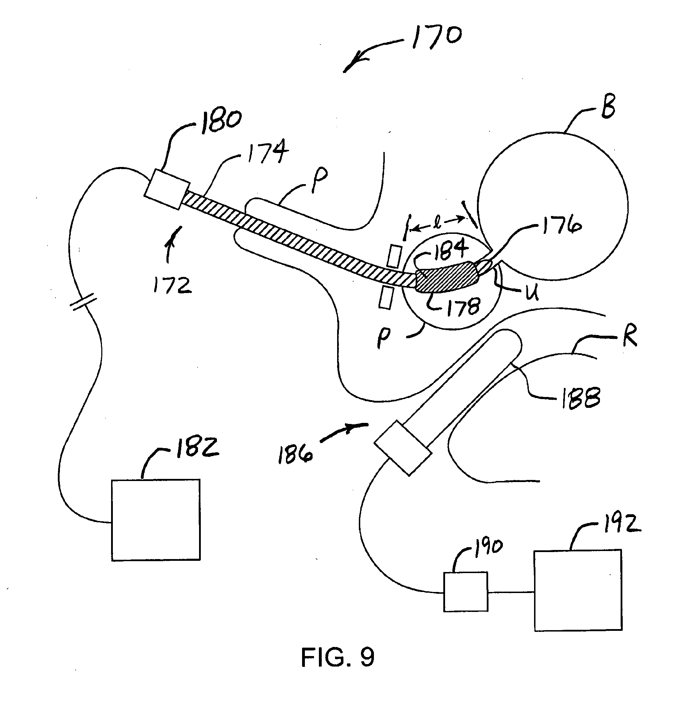

As noted above, access to the target tissue or prostate tissue can be gained through the urethra of the patient. Referring to FIG. 9, a urethral access system 170 according to the present invention is illustrated. The system 170 includes an elongated probe 172 that can be inserted in the urethra (U) of a patient via the penis (P), and advanced along the urethra (U) to the desired location within the patient's body, specifically at a target location in the prostate tissue or gland (P). The probe includes a flexible catheter having an elongated shaft 174 that can be bent or flexed while advanced into and through the urethra (U). The probe includes a distal tip 176, that can be shaped (e.g., rounded) to minimize damage or trauma to the urethral wall during positioning or use. The probe 172 can optionally include a drainage lumen (not shown) that allows fluid communication between an area distal to the distal tip and the exterior or a proximal portion of the probe 172, so as to allow draining or flushing of contents of the bladder (B) during treatment and use of the probe 172.

The urethral probe 172 includes a proximal end and a distal portion having an expandable member 178, such as a balloon configured for expansion in the urethra (U) of the patient. The proximal end is positioned outside the patient's body during use, and can include a hub or handle 180 that can be coupled to a controller or control unit, and power source 182. The expandable member 178 includes conductive electrode elements 184 patterned or disposed on an outer surface of the expandable member 178. The probe will include an elongated body 174 extending from the proximal portion of the device to the distal portion, and the elongated body can include an inner lumen or passage with electrical coupling members, such as insulated wires, for coupling the electrode elements 184 of the expandable member 178 to the proximal end and/or an externally positioned controller and/or power source 182. As indicated in Figure 9, the urethra of the patient (U) will include a length (1) passing through the prostate tissue (P) until reaching the bladder (B). The expandable member 178 of the probe 172 can include various shapes and configurations selected to span any portion of the length (1). The expandable member 178 can be configured to span the entire length (1) (or more) or may be sized to span less than the entire portion. The expandable member 178 may be positioned at any portion along the length (1) during treatment, as well as elsewhere along the patient's urethra (U), including portions at or adjacent to locations where the urethra (U) enters or exits the prostate tissue (P) area.

The probe will be designed to include electrode elements that can be positioned in the desired location and used for delivery of electric fields to the target tissue for treatment according to the present invention. Various embodiments of electrode elements can be included in the present invention and the probe can be designed or configured for delivery of electrical fields, for example, between the expandable member and opposing electrode(s) (e.g., secondary electrodes) positioned in or in the vicinity of the prostate tissue, with current fields in some embodiments established between electrodes and typically in a plurality of directions (e.g., radially) through a volume of tissue. Electrode elements 184 of the expandable member 178 can include conductive material deposited or patterned on a surface or at least a portion of the expandable member 178 that is brought into contact with the walls of the urethra (U) during treatment. In one embodiment, the expandable member 178 can be configured in a deployable configuration, such that the expandable member 178 may be positioned within the probe shaft and then deployed from the probe (e.g., from the distal end or tip of the probe) and expanded at the desired location. For example, the expandable member can be positioned or disposed within in the probe shaft or portion of the elongate body (e.g., shaft lumen) during advancement and positioning of the probe, and deployed from the probe once a desired position in the patient's urethra has been reached. Alternatively, in another embodiment, the expandable member or balloon (e.g., electrode patterned balloon) can be coupled and positioned along the length of the probe on an outer surface, with inflation or expansion of the expandable member controlled by an external pressure source coupled to the proximal portion of the probe.

A probe may include one or more electrodes (e.g., secondary electrodes) that can be positioned within the probe and deployed from the probe and into the prostate tissue. For example, such secondary electrodes can be positioned in the probe shaft or body during advancement and positioning of the probe, and deployed from the probe once a desired position has been reached. Deployable probes can include needle-like electrodes, which can include a shape memory metal and configured to assume a desired shape when deployed, e.g., as discussed further below.

During use, field delivery can occur with current flow between an electrode elements of the urethral probe and electrode(s) spaced from the urethral probe, such as electrodes positioned in the prostate tissue or in the rectal area. As above, electrode elements, including electrodes of the expandable member, will be connected to an external power source 182 or power unit (e.g., power source of control system or unit), which will include a means of generating electrical power for operation of the system and probe, and application of electrical current to the target tissue as described herein. The power unit can include or be operably coupled to additional components, such as a control unit, driver unit, user interface, and the like (see, e.g., infra).

System 170 further includes an imaging device 186, such as an ultrasonic imaging probe, for providing images of tissues for example during positioning and/or use of the probe 172. The device 186 includes a distal imaging portion 188 including electronics and imaging components (e.g., ultrasonic scanning transducer), which can be inserted in the patient's rectum (R) and positioned against the rectal wall near the prostate (P). Imaging device 186 can include those commonly used for diagnostic medicine (see, e.g, above). The imaging portion 188 can scan a region of the tissue to generate an image of the tissue, rectal wall, prostate (P), urethra (U), and/or the probe located in the patient's urethra (U). The imaging device 186 can be connected to an image processing unit 190 and a display unit 192, as is common practice. In use, the display 192 provides images (e.g., real-time ultrasonic images) of the prostate (P) with the position of the probe 172 relative to the prostate (P) and target area, the bladder (B), etc. to help guide or confirm positioning of the probe 172 within the prostate (P) prior to delivery of treatment energy.

As discussed above, a probe of a system, e.g., as illustrated in FIG. 9, will include electrode element 184 patterned or otherwise disposed on an expandable member or balloon 178 disposed on a distal portion of the probe 172. A probe can include a catheter probe having a shaft and a distally positioned balloon member having electrode elements disposed (e.g., deposited, patterned, etc.) thereon. The balloon can be coupled to one or more fluid sources positioned externally, as well as a pressure source and/or controller for inflation and deflation of the balloon. In one embodiment, the balloon can be configured such that a fluid can be circulated through the balloon and may be utilized to further effect or control temperature of tissues proximate to the balloon. The probe further includes a proximal hub 180 that can include one or more electrical connections for coupling the electrode elements to an external power source and/or control unit 182, as well as fluid connections for fluidic access and control of balloon actuation and inflation, as well as circulation of fluid (e.g., cooling fluid) through the balloon. In an embodiment where the probe 172 further includes one or more deployable electrodes, actuation and positioning of such deployable electrodes can be controlled from the proximal end of the probe, such as through the hub 180. In other embodiments, current flow can extend between electrode elements 184 of the expandable member 178 positioned in the patient's urethra (U) and one or more electrode elements (e.g., secondary electrodes) spaced from the positioned expandable member 178, and may be separate from the urethral probe, and positioned on an opposing side of the urethral wall. For example, needle electrodes can be separately advanced through the perineum of the patient and positioned within the prostate tissue (P) around the urethra (U), with energy delivery establishing current flow between electrode elements of the urethral probe and needle electrodes positioned in the prostate tissue (P). In yet another embodiment, electrode elements (e.g., electrodes disposed on an expandable balloon) can be positioned in the rectal cavity adjacent to the rectal wall, with current flow established between electrode elements of the urethral probe and electrode elements of the rectally positioned device.

In yet another aspect, access and delivery of the desired current may be gained through the rectal cavity. An energy delivery probe can be inserted into the rectum of a patient and electrodes positioned adjacent to the rectal wall or advanced through the rectal wall and into the prostate tissue of the patient. Various probe and/or electrode configurations may be suitable for delivery of the current in accordance with the present invention. A probe can include, for example, elongated device or catheter with one or more needle-like electrodes including, e.g., electrodes deployable from a catheter lumen. Alternatively, an energy delivery probe can include one or more inflatable devices or balloons having electrode patterns disposed on a surface. Such balloons may be similar to those described above with respect to transurethral access and current delivery. Rectal probes can be utilized in isolation, e.g., with electrodes of the rectal probe forming discrete energy delivery units (e.g., pairs or groups of bipolar electrodes), or electrodes of a rectal probe can operate in conjunction with other electrodes, such as electrodes of transuerethral probe or elongate electrodes inserted across the perineum of the patient. In the latter case, electrodes of the trans-rectal probe and separately positioned probe can be operated in bipolar mode such that current flow is established across tissue separating the different devices, and between the electrodes of the different devices.

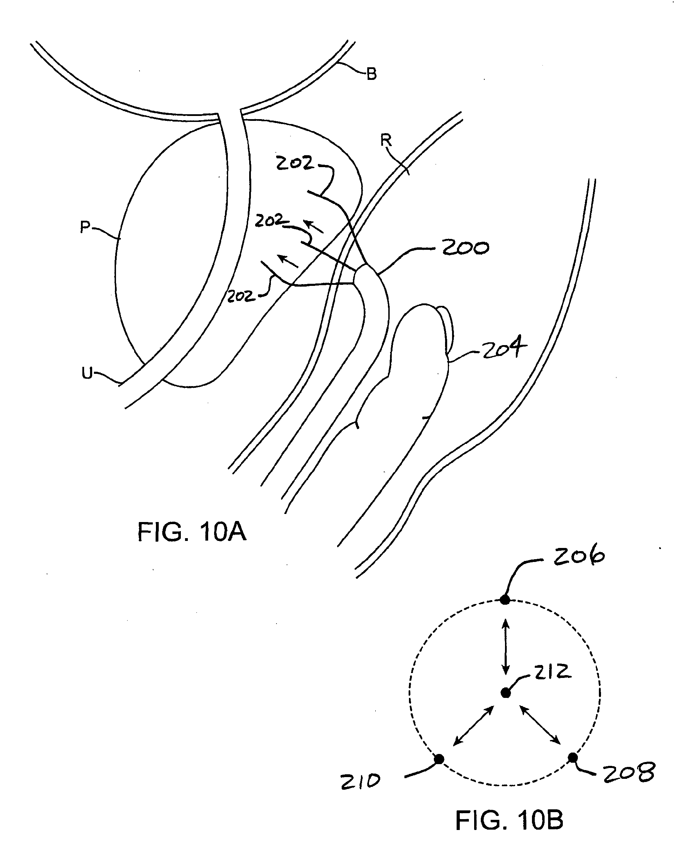

A trans-rectal approach, according to one embodiment of the present invention, is described with reference to Figures 10A and 10B. A current delivery probe 200 can be inserted into the rectal cavity (R) of a patient, and the probe and/or one or more electrodes 202 of the probe can be advanced through the rectal wall and into the prostate tissue (P). The probe 200 can include a catheter having an inner lumen, with one or more electrodes 202 deployable from a distal portion of the catheter. One advantage of such an approach is that the probe 200 can be more easily located or positioned at the desired location for advancement and delivery of electrode elements 202 to the desired location. A user or physician, for example, can access the rectal cavity and position the probe distal end by manipulation by hand or one or more fingers 204. The probe 200 can include a plurality of deployable electrodes 202 that can be positioned in the prostate tissue (P) so as to establish current flow in a plurality of different directions, such as with a radial field application. As shown in Figure 10B, for example, electrodes can include a plurality of outer electrodes 206, 208, 210 deployed and positioned to form or define an ablation volume, with an electrode 212 positioned within the volume. Current flow can be established between the centrally located electrode 212 and the outer electrodes 206, 208, 210 for radial field application and establishing current flow in a plurality of different directions. Treatment can further include use of an imaging device (not shown), such as an ultrasound imaging device as described above, which can be inserted and positioned in the rectal cavity (R) separately and/or in conjunction with other components (e.g., probe) of an energy delivery system of the present invention.

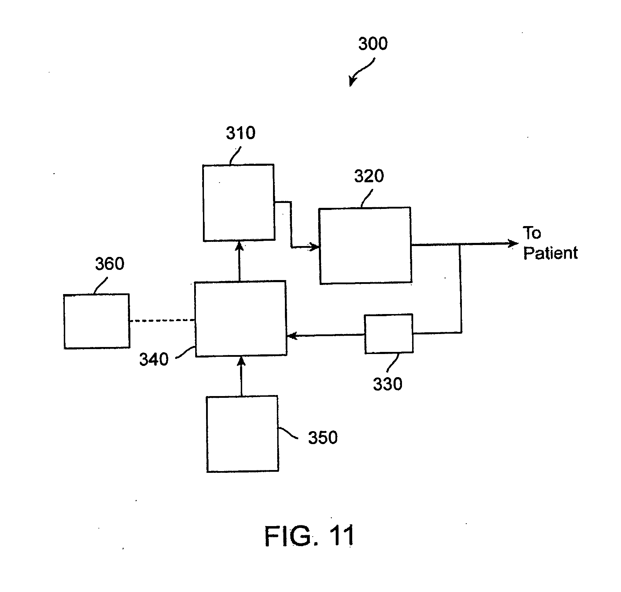

A system according to an embodiment of the present invention is described with reference to Figure 11. The system 300 can include incorporated therewith any device of the present invention for delivery of energy to the patient, and includes a power unit 310 that delivers energy to a driver unit 320 and than to electrode(s) of an inventive device. The components of the system individually or collectively, or in a combination of components, can comprise an energy source for a system of the invention. A power unit 310 can include any means of generating electrical power used for operating a device of the invention and applying electrical current to a target tissue as described herein. A power unit 310 can include, for example, one or more electrical generators, batteries (e.g., portable battery unit), and the like. One advantage of the systems of the present invention is the low power required for the ablation process. Thus, in one embodiment, a system of the invention can include a portable and/or battery operated device. A feedback unit 330 measures electric field delivery parameters and/or characteristics of the tissue of the target tissue region, measured parameters/characteristics including without limitation current, voltage, impedance, temperature, pH and the like. One or more sensors (e.g., temperature sensor, impedance sensor, thermocouple, etc.) can be included in the system and can be coupled with the device or system and/or separately positioned at or within the patient's tissue. These sensors and/or the feedback unit 330 can be used to monitor or control the delivery of energy to the tissue. The power unit 310 and/or other components of the system can be driven by a control unit 340, which may be coupled with a user interface 350 for input and/or control, for example, from a technician or physician. The control unit 340 and system 300 can be coupled with an imaging system 360 (see above) for locating and/or characterizing the target tissue region and/or location or positioning the device during use.

A control unit can include a, e.g., a computer or a wide variety of proprietary or commercially available computers or systems having one or more processing structures, a personal computer, and the like, with such systems often comprising data processing hardware and/or software configured to implement any one (or combination of) the method steps described herein. Any software will typically include machine readable code of programming instructions embodied in a tangible media such as a memory, a digital or optical recovering media, optical, electrical, or wireless telemetry signals, or the like, and one or more of these structures may also be used to transmit data and information between components of the system in any wide variety of distributed or centralized signal processing architectures.

Components of the system, including the controller, can be used to control the amount of power or electrical energy delivered to the target tissue. Energy may be delivered in a programmed or pre-determined amount or may begin as an initial setting with modifications to the electric field being made during the energy delivery and ablation process. In one embodiment, for example, the system can deliver energy in a "scanning mode", where electric field parameters, such as applied voltage and frequency, include delivery across a predetermined range. Feedback mechanisms can be used to monitor the electric field delivery in scanning mode and select from the delivery range parameters optimal for ablation of the tissue being targeted.

Systems and devices of the present invention can, though not necessarily, be used in conjunction with other systems, ablation systems, cancer treatment systems, such as drug delivery, local or systemic delivery, surgery, radiology or nuclear medicine systems, and the like. Another advantage of the present invention, is that treatment does not preclude follow-up treatment with other approaches, including conventional approaches such as surgery and radiation therapy. In some cases, treatment according to the present invention can occur in conjunction or combination with therapies such as chemotherapy. Similarly, devices can be modified to incorporate components and/or aspects of other systems, such as drug delivery systems, including drug delivery needles, electrodes, etc.

The following examples are intended to illustrate but not limit the invention.

### EXAMPLE

The present example describes a study designed to evaluate efficacy of different treatment parameters using the electric field delivery and ablation technology as described herein in the treatment of a human prostate cancer (CaP) xenograft model.

### Design

Sixty 4 to 6-week old male CB-17 SCID mice were injected subcutaneously on the right flank with 2*10⁶ cells of the C4-2B CaP cell line. After injection, animals enrolled once tumor volumes reached 200mm³ (∼3-4 weeks) and randomized into one of five groups using the following design: 1) Control group - received placement of probe without current (n=10); 2) a groups receiving 15 mAmp for 15 min (n=13); 3) a group receiving 15 mAmp for 60 min (n=9); 4) a group receiving 25 mAmp for 15 min (n=10); and 5) a group receiving 25 mAmp for 60 min (n=10). Mice were treated with direct application of a low power, intermediate frequency (e.g., about 100 kHz) field through percutaneous placement of a probe (e.g., as shown in Figures 2A-2C) in a fashion that affording the greatest tumor coverage. The day of treatment was designated as Day 1. The day prior to the treatment day was designated as Day -1.

A subsets of animals were sacrificed 7 days after treatment for histopathological evaluation of tumors. The remaining mice were sacrifice 14 days or more after treatment. Animals that had complete destruction of their tumors were observed for up to 30 days post treatment for recurrence. Tumor volumes were measured twice weekly and prostate specific antigen (PSA) levels were measured once a week.

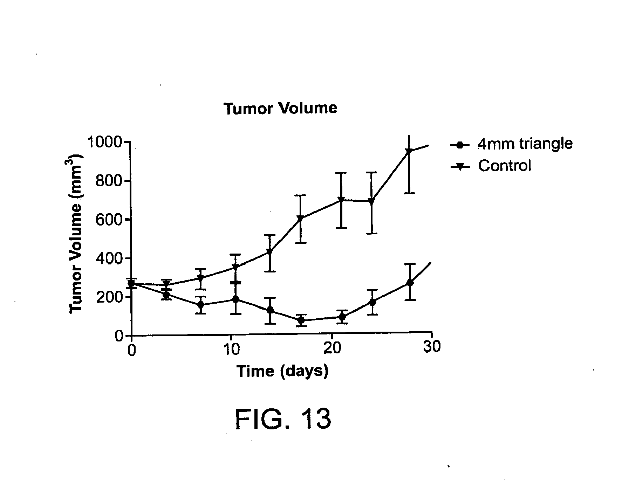

The probe used was of the triangle configuration with a central anode and three outer cathodes (see, e.g., Figures 2A-2D). The radius of the probe from anode to cathode was three millimeters in one example. A separate group was evaluated using a four millimeter anode to cathode spacing (see results illustrated in Figure 13). The electrode probe was coupled to a System Control Module (SCM) designed to generate, deliver, monitor and control the therapeutic field within the specified treatment parameters. The SCM included of an integrated direct current (DC) battery power source, an alternating current (AC) inverter, a signal generator, a signal amplifier, an oscilloscope, an operator interface monitor, and a central processing unit (CPU). The SCM was battery powered and isolated from ground. AC current was derived from the integrated power inverter. An intermediate frequency (about 100 kHz) alternating current, sinusoidal wave form signal can be produced from the signal generator. The signal is amplified to a current range of 5 mA to 40 mA and voltage of up to 20 Vrms. Total power output is less than 1 watt. Field characteristics including wave form, frequency, current and voltage are monitored by an integrated oscilloscope. Scope readings are displayed on the operator interface monitor. An integrated CPU monitors overall system power consumption and availability and controls the output of the signal generator and amplifier based on the treatment parameters input by the operator.

### Prostate Tumor Xenograft Model

The C4-2B CaP cell line was obtained and implanted. This is an castration-resistant CaP cell line derived from a bony metastasis of the LNCaP cell line. This line was maintained under standard conditions and propagated when necessary. Tumor measurement were done with hand held caliper begin once tumors become palpable and twice weekly thereafter.

Animals included CB-17 SCID male mice were obtained from Fox Chase SCID mice, Charles River, Wilmington, MA. Animals were eartagged and checked for health on arrival 11.07.07and group housed (five animals per cage) at the vivarium. Animals were acclimated to the facility for 7 days before beginning the experiment. Statistical analyses were performed using unpaired student t-tests and ANOVA (Prism Graphpad, Graphpad Software, San Diego, CA). Statistically significance results were designated as *P*≤0.05. After fixation, tumors were serially sectioned in 2-3mm increments from which 5 micron thick slides were cut and used for histopathology analysis.

### Dose Groups

Animals were randomly sorted and assigned into five different treatment groups (see Table 1) and randomized.

**Table 1: Treatment Groups and Animal Assignment**

| **Group** | **Animals (Numbers)** | **Treatment Conditions** | **Treatment Duration** | **Sacrifice Schedule (Animal Number)** | |
|---|---|---|---|---|---|
| | | | | **Day 7** | **Day 14** |
| 1 | 12 | 0 mAmp | XX min | 1-6 | 7-12 |
| 2 | 12 | 15 mAmp | 15 min | 13-18 | 19-24 |
| 3 | 12 | 15 mAmp | 60 min | 25-30 | 30-36 |
| 4 | 12 | 25 mAmp | 15 min | 37-42 | 43-48 |
| 5 | 12 | 25 mAmp | 60 min | 49-54 | 55-60 |

All animals were closely observed daily for signs of lethargy, weight loss, paralysis, dyspnea, cyanosis, mucopurulent discharges, incontinence, diarrhea, changes in coat or body condition, or any other health problems that could indicate that the animal was becoming moribund (as defined by IACUC guidelines). All observations were documented and members of the research staff were notified if any abnormalities were found. Any animal found with apparent health problems was monitored at additional times, as needed. Any animal appearing moribund was promptly euthanized.

Mice were bleed (∼20uL) from the tail vein once weekly starting with enrollment. Serum was removed after centrifugation for 8 min at 10000 RPM. PSA levels were then determined using IMx Total PSA Assay, Abbott Laboratories, Abbott Park, IL. Intra-tumoral temperature measurements were made using thermocouples positioned in the tissue. Baseline temperature measurements were taken prior to application of power and at 15 minute intervals. Current delivery was selected to avoid damage due to severe temperature elevation (e.g., exceeding 50 degrees C).

### RESULTS

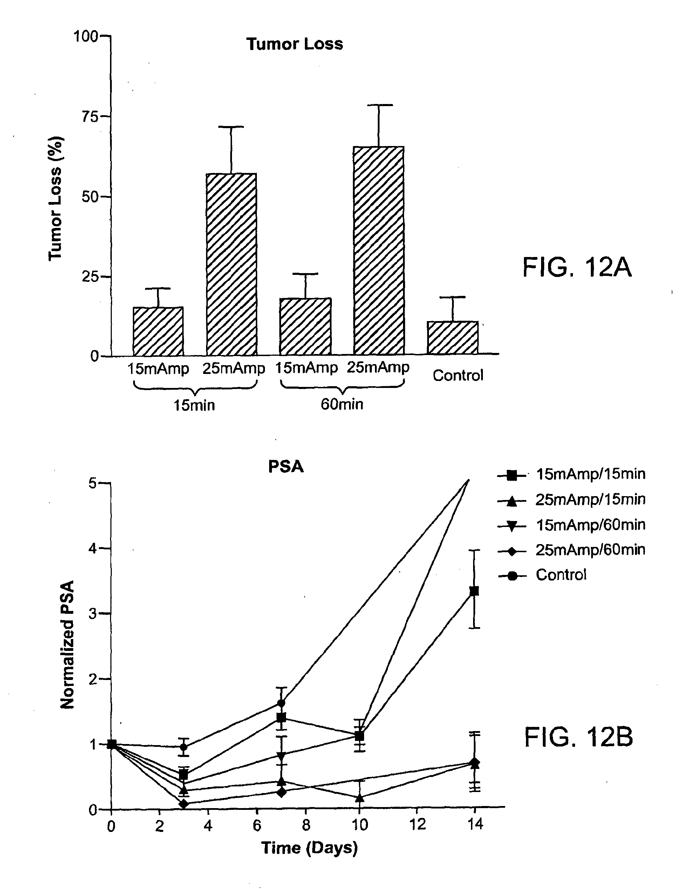

Animals tolerated the procedure well with no observable adverse side effectsattributed to the application of the treatment. The animals that received 15 mAmp of current applied to their tumors demonstrated a 17 ± 4.7% (mean ± SEM) decrease in enrollment tumor volume at the lowest nadir following treatment. These reductions were greater than (though not significantly different) from those seen in the control group (Control - 10 ± 6.9%, *p=0*.*436*). When comparing the groups receiving 15 mAmp/15 min vs. 15 mAmp/60 min there was no significant difference to tumor volume reductions (*p*= *0.85*). The animals that had 25mAmp of current applied to their tumors had a 62 ± 9.4% decrease in tumor volume at their lowest nadir. This is a significant decrease in tumor volume compared to both the control group (*p=0.001*) and 15 mAmp treated animals (*p<0. 001*). There were no differences in tumor volume reduction measured between the group receiving 25 mAmp for 15 min and the group receiving 25 mAmp for 60 min (*p*=*0.704*). It was noted that 6/20 animals treated with 25 mAmp demonstrated a complete ablation/destruction of the tumor. Results of treatment on tumor volume are illustrated in Figure 12A. Results of treatment on tumor volume using the four millimeter probe compared to control is shown in Figure 13. Using the 4 mm probe configuration with 33 mAmp treatment, approximately half of the tested animals had complete ablation/destruction of the tumor.

### Prostate-Specific Antigen

PSA levels generally tracked well with treatment effectiveness and tumor volume reductions. PSA levels normalized to enrollment levels are shown in Figure 12B. Normalized levels were examined at 14 days from enrollment. Some non-statistically significant reduction was seen in PSA levels in the 15 mAmp treated animals compared to control (4.4 ± 1.1 vs. 6.1 ± 4.3, *p*=*0.634*). The normalized PSA levels in the 25 mAmp treated at 14 days was 0.67± 0.3 which represents a significant reduction compared to the 15 mAmp treated animals(*p*=*0.005)*. Due to the large variation in the control group PSA levels, no statistical difference could be found between the 25 mAmp animals and control animals (though trend differences were observed). No significant differences in PSA levels were measured between groups receiving the same current but a different time intervals.

### TissueTemperature

Intra-tumoral temperatures were measured immediately prior to and during each treatment in most study groups. Animal body temperature was typically around 37°C. Tumor tissue temperature of animals under anesthesia dropped below normal average body temperature. During treatment, the 15 mAmp treatment groups rose to a maximum temperature of 36 ± 0.6°C (mean ± SEM). This represents a 6.5 ± 1.1°C elevation above baseline temperatures during treatment. The maximum temperature in the 25 mAmp treatments groups was significantly higher compared to the 15 mAmp treated groups (25 mAmp: 44 ± 0.6°C; *p<0.001*) with significantly higher elevations in temperature above baseline vs. 15 mAmp treated groups (15 ± 0.6°C; *p*=*<0*.*001*).

The described low-power, mild hyperthermia treatment demonstrated significant tumoricidal capabilities. The results show that efficacy is based on the current applied and with effective treatment occurring in shortest tested treatment times. Tissue heating due to treatment was limited to average treatment temperatures of about 44°C, which would seem to preclude as a cytotoxic factor effects of more extreme temperature application characterized by tissue charring and substantial protein cross-linking typically observed at temperatures well in excess of 50°C.

Further, elevations in temperature to this level have typically required far greater lengths of treatment than the observed treatment times shown to have effectiveness in this study. It is possible that both the elevations in temperature along with factors such as the application of alternating electrical current and/or field orientation cumulatively or synergistically allow for shorter time intervals necessary to derive at the desired tumor ablating effect.

It is further noted that further refinements and/or customization of delivery probes or positioned electrodes to individual tumors being treated may further improve treatment results. In some subjects, electrodes did not encompass the entire tumor or in some cases were entirely contained within the tumor margin and, therefore, less than the entire tumor was treated in such instances. Complete tumor destruction was seen in some animals and was observed more likely in instances where the tumor was more thoroughly contained within the treatment volume. Further, as a group, improved results were observed in the study group using the larger sized probe with 4 mm anode/cathode spacing, where on average tumors were more completely treated.

It is understood that the examples and embodiments described herein are for illustrative purposes only and that various modifications or changes in light thereof will be suggested to persons skilled in the art and are to be included within the purview of this application and scope of the appended claims. Numerous different combinations are possible, and such combinations are considered part of the present invention.

## Claims

1. A system for preferential destruction of cancerous or hyperplastic cells of a prostate tissue of a patient, comprising:
a plurality of electrodes (22) for advancement and positioning in a target tissue region of the patient comprising prostate tissue,;
a control system (84) comprising a power source coupled to the electrodes (22), and a computer readable storage media comprising instructions that, when executed, cause the control system (84) to:
provide electrical current to the electrodes (22);
charcterised in that
each of the plurality of electrodes (22) has a current field delivery region, one or more of the electrodes (22) being configured to be advanced through tissue to position the current field delivery region of the respective electrode within the prostrate tissue: and
the computer readable storage media comprises instructions that, when executed, cause the control system (84) provide electrical current to the electrodes (22) so as to deliver electrical current via the current field delivery regions to establish a current flow radially or in a plurality of different directions through a volume of the prostate tissue and to preferentially destroy cancerous or hyperplastic cells in the target tissue region and
the computer readable storage media comprises instructions that, when executed, cause the control system (84) to maintain for a period of treatment an average target tissue temperature of less than 50 degrees C during energy delivery

2. The system of claim 1, wherein the plurality of electrodes (22) comprises three or more secondary electrodes (34, 36, 38) positioned to at least partially define an ablation volume and a center electrode (32)within the ablation volume, the electrodes (22) positioned such that during energy application a current flow field is established between a center electrode (32) and the secondary electrodes (34, 36, 38) extending radially outward from a current flow center,
and wherein the plurality of electrodes (22) are coupled to a housing of a probe in a fixed position.

3. The system of claim 2, further comprising an elongate urethral probe (172), comprising a proximal end and a distal portion having an expandable member (178), one or more central electrodes (22) being disposed on an outer surface of the expandable member (178), so that, in use the expandable member (178) can be expanded at a target location so as to position the one or more central electrodes (22) in contact with an inner surface of a patient's urethra,
wherein one or more of the secondary electrodes (22) are deployable from a body of the elongate probe (172),and wherein the secondary electrodes (22) comprise elongate needle electrodes (22).

4. The system of claim 3, further comprising heated or cooled inflation media, which, in use, is flowed through the expandable member.

5. The system of claim 1, or 2 for preferential destruction of cancerous cells or hyperplastic tissues of the prostate, wherein the plurality of electrodes (22) comprises an array of needle electrodes (22) which, in use, are advanced through the perineum of a patient to a target tissue region, the array being such that groups of electrodes (22) can be differentially activated so as, in use, to establish an electrical current flow radially or in a plurality of different directions through the tissue.

6. The system of claim 5, further comprising a template device (100) having guides (106) through which, in use, the electrodes (22) may be advanced to position them.

7. The system of claim 5 or 6, wherein two or more groups of electrodes (22) can be differentially activated in seriatim.

8. The system of any preceding claim, further comprising a feedback unit (330) for detecting temperature in the target tissue region, preferably wherein the control system (84) and feedback unit (330) are coupled so as to maintain an average target tissue region temperature less than 50 degrees C or from 40 degrees C to 48 degrees C during treatment comprising energy delivery, and/or an imaging system (360).

9. The system of any preceding claim for use in a method of delivering electric fields to a prostate tissue of a patient, the method comprising:
positioning the plurality of electrodes (22) in a target tissue region comprising the prostate tissue; and
establishing an the electrical current flow radially or in a plurality of different directions through a volume of the prostate tissue so as to preferentially ablate cancerous cells in the volume, the electrical current flow comprising a frequency of less than about 300 kHz.

10. The system of claim 9, wherein establishing the current flow comprises applying an alternating electrical current to the volume to provide an electric field extending radially outward from a current flow center, and wherein the system comprises a plurality of electrodes (22) positionable to define an ablation volume such that, in use, the electric field extends radially outward from a current flow center established by the plurality of electrodes (22), with electrodes (22), positioned around the volume, activated in pairs to establish current flow between electrodes (22).

11. The system of claim 9 or 10, wherein the method further comprises positioning a plurality of secondary electrodes (34, 36, 38) to at least partially define an ablation volume comprising the target tissue, positioning a center electrode (32) within the ablation volume, and establishing a current flow field between the center electrode (32) and the secondary electrodes (34, 36, 38) extending radially outward from a current flow center.

12. The system of claim -9, 10 or 11, wherein positioning the electrodes (22) comprises advancing a probe (200) comprising a plurality of electrodes (22) to the target tissue region, preferably wherein the probe (200) is advanced through a surgical incision, a percutaneous puncture, a perineal, a transurethral, or transrectal access procedure, and/or wherein the positioning comprises positioning electrodes (22) in the prostate tissue and adjacent to or around the urethra of the patient.

13. The system of any one of claims 9 to 12, wherein the method further comprises surgically removing at least a portion of the prostate or performing a brachytherapy procedure following electric field delivery monitoring a patient's PSA level following electric field delivery to detect prostate tissue ablation.

14. The system of any one of claims 9 to 13, wherein the method further comprises delivering alternating electrical current substantially throughout the volume of the patient's prostate during treatment, the alternating current flow comprising a frequency of 50 to 300 kHz or 50 to 250 kHz or 100 kHz and/or maintaining for a period of treatment an average target tissue region temperature below 50 degrees C or from 40 degrees C to 48 degrees C or from 42 degrees C to 45 degrees C.

15. The system of any preceding claim, wherein at least one of the electrodes (22) is or includes one or more of: (a) a needle electrode, (b) a deployable element, (c) curved, and (d) substantially straight or linear.

## Patentansprüche

1. System zur bevorzugten Zerstörung von Krebs- oder hyperplastischen Zellen eines Prostatagewebes eines Patienten, umfassend:
eine Mehrzahl von Elektroden (22), die vorwärts bewegt und in einem Zielgewebebereich des Prostatagewebes des Patienten positioniert werden;
ein Steuerungssystem (84) umfassend eine mit den Elektroden (22) verbundene Stromquelle und ein computerlesbares Speichermedium umfassend Anweisungen, die beim Ausführen bewirken, dass das Steuerungssystem (84):
den Elektroden (22) elektrischen Strom bereitstellt;
**dadurch gekennzeichnet, dass**
jede der Mehrzahl von Elektroden (22) einen Stromfeld-Zuführungsbereich aufweist, wobei eine oder mehr der Elektroden (22) konfiguriert sind, durch Gewebe hindurch vorwärts bewegt zu werden, um den Stromfeld-Zuführungsbereich der jeweiligen Elektrode innerhalb des Prostatagewebes zu positionieren, und
das computerlesbare Speichermedium Anweisungen umfasst, die beim Ausführen bewirken, dass das Steuerungssystem (84) den Elektroden (22) elektrischen Strom bereitstellt, um elektrischen Strom über die Stromfeld-Zuführungsbereiche zuzuführen, um einen Stromfluss radial oder in einer Mehrzahl von verschiedenen Richtungen durch ein Volumen des Prostatagewebes hindurch aufzubauen und um bevorzugt Krebs- oder hyperplastische Zellen im Zielgewebebereich zu zerstören, und
das computerlesbare Speichermedium Anweisungen umfasst, die beim Ausführen bewirken, dass das Steuerungssystem (84) für einen Behandlungszeitraum eine durchschnittliche Zielgewebetemperatur von weniger als 50 Grad C während der Energiezuführung aufrechterhält.

2. System nach Anspruch 1, worin die Mehrzahl von Elektroden (22) drei oder mehr sekundäre Elektroden (34, 36, 38) umfasst, die positioniert sind, um zumindest teilweise ein Ablationsvolumen zu definieren, und eine mittlere Elektrode (32) innerhalb des Ablationsvolumens, wobei die Elektroden (22) so positioniert sind, dass beim Anlegen von Energie ein von einer Stromflussmitte radial nach außen verlaufendes Stromflussfeld zwischen einer mittleren Elektrode (32) und den sekundären Elektroden (34, 36, 38) aufgebaut wird,
und worin die Mehrzahl von Elektroden (22) mit einem Gehäuse einer Sonde in einer festen Position verbunden ist.

3. System nach Anspruch 2, ferner umfassend eine längliche Harnröhrensonde (172) umfassend ein proximales Ende und einen distalen Teil mit einem erweiterbaren Element (178), wobei eine oder mehr mittlere Elektroden (22) auf einer Außenfläche des erweiterbaren Elements (178) angeordnet sind, sodass das erweiterbare Element (178) bei Gebrauch an einem Zielort erweitert werden kann, um die eine oder mehr mittleren Elektroden (22) eine innere Oberfläche der Harnröhre eines Patienten berührend zu positionieren,
worin eine oder mehr der sekundären Elektroden (22) von einem Körper der länglichen Probe (172) aus einsetzbar sind und worin die sekundären Elektroden (22) längliche Nadelelektroden (22) umfassen.

4. System nach Anspruch 3, ferner umfassend ein erwärmtes oder gekühltes Aufblasmittel, das bei Gebrauch durch das erweiterbare Element hindurch fließt.

5. System nach Anspruch 1 oder 2 zur bevorzugten Zerstörung von Krebszellen oder hyperplastischen Geweben der Prostata, worin die Mehrzahl von Elektroden (22) ein Array von Nadelelektroden (22) umfasst, die bei Gebrauch durch das Perineum eines Patienten hindurch zu einem Zielgewebebereich vorwärts bewegt werden, wobei das Array so ausgeführt ist, dass Gruppen von Elektroden (22) differentiell aktiviert werden können, um bei Gebrauch einen elektrischen Stromfluss radial oder in einer Mehrzahl von verschiedenen Richtungen durch das Gewebe hindurch aufzubauen.

6. System nach Anspruch 5, ferner umfassend eine Schablonenvorrichtung (100) mit Führungen (106), durch welche hindurch die Elektroden (22) bei Gebrauch zu deren Positionierung vorwärts bewegt werden können.

7. System nach Anspruch 5 oder 6, worin zwei oder mehr Gruppen von Elektroden (22) der Reihe nach differentiell aktiviert werden können.

8. System nach irgendeinem vorhergehenden Anspruch, ferner umfassend eine Rückmeldeeinheit (330) zum Feststellen der Temperatur im Zielgewebebereich, vorzugsweise worin das Steuerungssystem (84) und die Rückmeldeeinheit (330) verbunden sind, um eine durchschnittliche Zielgewebebereichstemperatur von weniger als 50 Grad C oder von 40 Grad C bis 48 Grad C während der Behandlung umfassend Energiezuführung aufrechtzuerhalten, und/oder ein Bildgebungssystem (360).

9. System nach irgendeinem vorhergehenden Anspruch zur Verwendung bei einem Verfahren zur Zuführung elektrischer Felder zu einem Prostatagewebe eines Patienten, wobei das Verfahren Folgendes umfasst:
Positionieren der Mehrzahl von Elektroden (22) in einem Zielgewebebereich umfassend das Prostatagewebe; und
Aufbauen eines elektrischen Stromflusses radial oder in einer Mehrzahl von verschiedenen Richtungen durch ein Volumen des Prostatagewebes hindurch, um bevorzugt Krebszellen in dem Volumen zu ablatieren, wobei der elektrische Stromfluss eine Frequenz von weniger als etwa 300 kHz umfasst.

10. System nach Anspruch 9, worin das Aufbauen des Stromflusses das Anlegen eines elektrischen Wechselstroms an das Volumen umfasst, um ein elektrisches Feld bereitzustellen, das radial von einer Stromflussmitte nach außen verläuft, und worin das System eine Mehrzahl von Elektroden (22) umfasst, die positionierbar sind, um ein Ablationsvolumen zu definieren, sodass, bei Gebrauch, das elektrische Feld von einer durch die Mehrzahl von Elektroden (22) aufgebauten Stromflussmitte radial nach außen verläuft, wobei um das Volumen herum positionierte Elektroden (22) paarweise aktiviert werden, um Stromfluss zwischen Elektroden (22) aufzubauen.

11. System nach Anspruch 9 oder 10, worin das Verfahren ferner das Positionieren einer Mehrzahl von sekundären Elektroden (34, 36, 38), um zumindest teilweise ein das Zielgewebe umfassendes Ablationsvolumen zu definieren, das Positionieren einer mittleren Elektrode (32) innerhalb des Ablationsvolumens und das Aufbauen eines von einer Stromflussmitte radial nach außen verlaufenden Stromflussfelds zwischen der mittleren Elektrode (32) und den sekundären Elektroden (34, 36, 38) umfasst.

12. System nach Anspruch 9, 10 oder 11, worin das Positionieren der Elektroden (22) das Vorwärtsbewegen einer Sonde (200) umfassend eine Mehrzahl von Elektroden (22) zum Zielgewebebereich umfasst, vorzugsweise worin die Sonde (200) durch eine chirurgische Inzision, eine perkutane Punktion, einen perinealen, einen transurethralen oder transrektalen Zugangseingriff hindurch vorwärts bewegt wird, und/oder worin das Positionieren das Positionieren von Elektroden (22) im Prostatagewebe und angrenzend an die Harnröhre des Patienten oder um die Harnröhre des Patienten herum umfasst.

13. System nach irgendeinem Anspruch 9 bis 12, worin das Verfahren ferner das operative Entfernen zumindest eines Teils der Prostata oder das Durchführen eines Brachytherapie-Eingriffs im Anschluss an die Zuführung eines elektrischen Felds oder die Überwachung des PSA-Spiegels eines Patienten im Anschluss an die Zuführung eines elektrischen Felds zur Feststellung der Prostatagewebe-Ablation umfasst.

14. System nach irgendeinem Anspruch 9 bis 13, worin das Verfahren ferner das Zuführen von elektrischem Wechselstrom im Wesentlichen im gesamten Volumen der Prostata des Patienten während der Behandlung umfasst, wobei der Wechselstromfluss eine Frequenz von 50 bis 300 kHz oder 50 bis 250 kHz oder 100 kHz und/oder das Aufrechterhalten, für einen Behandlungszeitraum, einer durchschnittlichem Zielgewebebereichstemperatur unterhalb von 50 Grad C oder von 40 Grad C bis 48 Grad C oder von 42 Grad C bis 45 Grad C umfasst.

15. System nach irgendeinem vorhergehenden Anspruch, worin zumindest eine der Elektroden (22) eine oder mehrere von folgenden Möglichkeiten ist oder beinhaltet: (a) eine Nadelelektrode, (b) ein einsetzbares Element, (c) gekrümmt und (d) im Wesentlichen gerade oder linear.

## Revendications

1. Système pour la destruction préférentielle de cellules cancéreuses ou hyperplasiques d'un tissu de prostate d'un patient, comprenant :
une pluralité d'électrodes (22) pour l'avance et le positionnement dans une région de tissu cible du patient comprenant un tissu de prostate ;
un système de commande (84) comprenant une source électrique couplée aux électrodes (22), et un support de stockage lisible par ordinateur comprenant des instructions qui, lorsqu'elles sont exécutées, amènent le système de commande (84) à :
fournir un courant électrique aux électrodes (22) ;
**caractérisé en ce que** :
chaque électrode de la pluralité d'électrodes (22) comporte une région de fourniture de champ de courant, une ou plusieurs des électrodes (22) étant conçues pour être avancées à travers le tissu afin de positionner la région de fourniture de champ de courant de l'électrode respective dans le tissu de prostate ; et
le support de stockage lisible par ordinateur comprend des instructions qui, lorsqu'elles sont exécutées, amènent le système de commande (84) à fournir un courant électrique aux électrodes (22) de manière à fournir un courant électrique via les régions de fourniture de champ de courant afin d'établir un flux de courant radialement ou dans une pluralité de directions différentes à travers un volume du tissu de prostate, et afin de détruire préférentiellement des cellules cancéreuses ou hyperplasiques dans la région de tissu cible ; et
le support de stockage lisible par ordinateur comprend des instructions qui, lorsqu'elles sont exécutées, amènent le système de commande (84) à maintenir pendant une période de traitement une température moyenne de tissu cible inférieure à 50 degrés C pendant la fourniture d'énergie.

2. Système selon la revendication 1, dans lequel la pluralité d'électrodes (22) comprend au moins trois électrodes secondaires (34, 36, 38) positionnées de manière à définir au moins partiellement un volume d'ablation, et une électrode centrale (32) dans le volume d'ablation, les électrodes (22) étant positionnées de sorte que pendant l'application d'énergie, un champ de flux de courant soit établi entre l'électrode centrale (32) et les électrodes secondaires (34, 36, 38) s'étendant radialement vers l'extérieur à partir d'un centre de flux de courant,
et dans lequel la pluralité d'électrodes (22) est couplée à un boîtier d'une sonde dans une position fixe.

3. Système selon la revendication 2, comprenant en outre une sonde urétrale allongée (172), comprenant une extrémité proximale et une partie distale comportant un élément dilatable (178), une ou plusieurs électrodes centrales (22) étant disposées sur une surface extérieure de l'élément dilatable (178), de telle sorte que, lors de l'utilisation, l'élément dilatable (178) puisse être dilaté au niveau d'un emplacement cible de manière à positionner la ou les électrodes centrales (22) en contact avec une surface intérieure de l'urètre d'un patient,
dans lequel une ou plusieurs des électrodes secondaires (22) sont déployables à partir d'un corps de la sonde allongée (172), et dans lequel les électrodes secondaires (22) comprennent des électrodes-aiguilles allongées (22).

4. Système selon la revendication 3, comprenant en outre un support de gonflage chauffé ou refroidi qui, lors de l'utilisation, est mis en circulation à travers l'élément dilatable.

5. Système selon la revendication 1 ou 2 pour la destruction préférentielle de cellules cancéreuses ou de tissus hyperplasiques de la prostate, dans lequel la pluralité d'électrodes (22) comprend un réseau d'électrodes-aiguilles (22) qui, lors de l'utilisation, sont avancées à travers le périnée d'un patient vers une région de tissu cible, le réseau étant tel que des groupes d'électrodes (22) peuvent être activés différentiellement de manière, lors de l'utilisation, à établir un flux de courant électrique radialement ou dans une pluralité de directions différentes à travers le tissu.

6. Système selon la revendication 5, comprenant en outre un dispositif de gabarit (100) comportant des guides (106) à travers lesquels, lors de l'utilisation, les électrodes (22) peuvent être avancées en vue de leur positionnement.

7. Système selon la revendication 5 ou 6, dans lequel au moins deux groupes d'électrodes (22) peuvent être activés différentiellement l'un après l'autre.

8. Système selon l'une quelconque des revendications précédentes, comprenant en outre une unité de rétroaction (330) destinée à détecter la température dans la région de tissu cible, de préférence dans lequel le système de commande (84) et l'unité de rétroaction (330) sont couplés de manière à maintenir une température moyenne de région de tissu cible inférieure à 50 degrés C ou entre 40 degrés C et 48 degrés C pendant le traitement consistant à fournir de l'énergie, et/ou comprenant un système d'imagerie (360).

9. Système selon l'une quelconque des revendications précédentes destiné à être utilisé dans un procédé de fourniture de champs électriques à un tissu de prostate d'un patient, le procédé consistant à :
positionner la pluralité d'électrodes (22) dans une région de tissu cible comprenant le tissu de prostate ; et
établir un flux électrique radialement ou dans une pluralité de directions différentes à travers un volume du tissu de prostate, de manière, de préférence, à procéder à l'ablation des cellules cancéreuses dans le volume, le flux de courant électrique ayant une fréquence inférieure à environ 300 kHz.

10. Système selon la revendication 9, dans lequel l'établissement du flux de courant consiste à appliquer un courant électrique alternatif au volume afin de fournir un champ électrique s'étendant radialement vers l'extérieur à partir d'un centre de flux de courant, le système comprenant une pluralité d'électrodes (22) positionnables de manière à définir un volume d'ablation de telle sorte que, lors de l'utilisation, le champ électrique s'étende radialement vers l'extérieur à partir d'un centre de flux de courant établi par la pluralité d'électrodes (22), les électrodes (22), positionnées autour du volume, étant activées par paires afin d'établir le flux de courant entre les électrodes (22).

11. Système selon la revendication 9 ou 10, dans lequel le procédé consiste en outre à positionner une pluralité d'électrodes secondaires (34, 36, 38) de manière à définir au moins partiellement un volume d'ablation comprenant le tissu cible, positionner une électrode centrale (32) dans le volume d'ablation, et établir un champ de flux de courant entre l'électrode centrale (32) et les électrodes secondaires (34, 36, 38) s'étendant radialement vers l'extérieur à partir d'un centre de flux de courant.

12. Système selon la revendication 9, 10 ou 11, dans lequel le positionnement des électrodes (22) consiste à faire avancer une sonde (200) comprenant une pluralité d'électrodes (22) vers la région de tissu cible, de préférence dans lequel la sonde (200) est avancée à travers une incision chirurgicale, une ponction percutanée ou une procédure d'accès périnéale, transurétrale ou transrectale, et/ou dans lequel le positionnement consiste à positionner les électrodes (22) dans le tissu de prostate et à proximité ou autour de l'urètre du patient.

13. Système selon l'une quelconque des revendications 9 à 12, dans lequel procédé consiste en outre à retirer chirurgiquement au moins une partie de la prostate ou à réaliser une procédure de curiethérapie après la fourniture d'un champ électrique surveillant le niveau de PSA d'un patient après la fourniture d'un champ électrique destiné à détecter l'ablation d'un tissu de prostate.

14. Système selon l'une quelconque des revendications 9 à 13, dans lequel le procédé consiste en outre à fournir un courant électrique alternatif essentiellement dans tout le volume de la prostate du patient pendant le traitement, le flux de courant alternatif ayant une fréquence de 50 à 300 kHz ou de 50 à 250 kHz ou de 100 kHz et/ou à maintenir pendant une période de traitement une température moyenne de région de tissu cible inférieure à 50 degrés C ou entre 40 degrés C et 48 degrés C ou entre 42 degrés C et 45 degrés C.

15. Système selon l'une quelconque des revendications précédentes, dans lequel au moins une des électrodes (22) consiste en ou comprend un ou plusieurs des éléments suivants : (a) une électrode-aiguille, (b) un élément déployable (c) une électrode incurvée et (d) une électrode essentiellement droite ou linéaire.
